# EUROPEAN PATENT APPLICATION

(11) **EP 4 682 252 A1**
(43) Date of publication of application: **21.01.2026**
(21) Application number: 24771011.4
(22) Date of filing: 15.03.2024
(51) Int. Cl.: C12N 7/01, A01N 63/40, A01P 1/00, A01P 3/00, A61K 35/76, A61P 31/04, C12N 15/55, C12N 15/113

(54) **RECOMBINANT PHAGE AND METHOD FOR PREPARING SAME**

(30) Priority: 15.03.2023 JP 2023041423
(71) Applicant: Japan Institute for Health Security, Tokyo 162-8655 (JP)
(72) Inventor: AZAM, Aa Haeruman, Tokyo 162-8655 (JP); KIGA Kotaro, Tokyo 162-8655 (JP); WATASHI Koichi, Tokyo 162-8655 (JP); TAKAHASHI Yoshimasa, Tokyo 162-8655 (JP)
(74) Representative: Ipsilon
(86) International application number: PCT/JP2024/010325
(87) International publication number: WO 2024/190909

(57) **Abstract**

An object of the present invention is to provide a recombinant phage and a method for preparing the recombinant phage, which can inactivate an anti-phage defense system of a bacterium including an antimicrobial-resistant bacterium, infect the bacterium, proliferate in the bacterium, and finally kill the bacterium. The present invention relates to a recombinant phage containing at least one of a gene encoding a Retron inhibitor and a gene encoding a tRNA functioning in a target bacterium, and a method for preparing the recombinant phage.

## Description

### TECHNICAL FIELD

The present invention relates to a recombinant phage and a method for preparing the recombinant phage.

### BACKGROUND ART

Antibacterial drugs have played the most important role in the treatment of bacterial infections. However, due to the appearance and rapid spread of antimicrobial-resistant bacteria for which current antibacterial drugs are not effective, the existing antibacterial drugs have become ineffective, and bacterial infections have once again become a worldwide problem threatening human health. This problem is serious in that antimicrobial-resistant bacteria appear at a speed far higher than that of the development of antibacterial drugs, and the development in novel antibacterial drugs has been blocked. That is, it is difficult to solve the problem of antimicrobial-resistant bacteria in an antibacterial drug development strategy according to the related art.

In recent years, an antibacterial treatment method (phage therapy) using bacteriophage (hereinafter abbreviated as phage), which is a sterilization mechanism different from that of an antibacterial drug according to the related art, has attracted attention, and clinical trials have been conducted mainly in Europe and the United States. However, when an antimicrobial-resistant bacterium derived from a clinically isolated strain is actually infected with a phage, the phage is repelled with a considerable probability. Therefore, it is not easy to collect a phage capable of strongly infecting the antimicrobial-resistant bacterium.

This is because the antimicrobial-resistant bacteria harbor some anti-phage defense systems. The anti-phage defense system refers to a defense mechanism like an immune system of bacteria against phages, and examples thereof include Argonaute (RNAi in nematodes, plants, and animals), CRISPR-Cas, Retron (Non-Patent Literature 1), and restriction enzyme systems. Only phages that have circumvented these anti-phage defense systems can infect antimicrobial-resistant bacteria. That is, for phage therapy to succeed, the phage needs to have a function of inactivating the anti-phage defense system of antimicrobial-resistant bacteria. Therefore, a phage capable of inactivating the anti-phage defense system of antimicrobial-resistant bacteria has been collected each time and provided for the phage therapy. However, this is a technique that is left to chance. It is not easy to collect effective phages for antimicrobial-resistant bacteria having diversity, and a great deal of effort is required.

Specifically, for example, a bactericidal composition containing a carrier and at least one type of phage, in which a phage having a sufficiently high concentration for inducing lysis-from-without by the phage is provided in the composition, has been studied (Patent Literature 1).

However, in the case where the bactericidal composition is used, it is necessary to add a composition at a high concentration to a pathogenic bacterium, which is mainly a target of sterilization, among the bacteria. This method is also a method that is left to chance because only phages which accidentally break an anti-phage defense system of bacteria proliferate inside the bacteria, and it is not easy to collect effective phages for antimicrobial-resistant bacteria having diversity.

### CITATION LIST

### PATENT LITERATURE

Patent Literature 1: JP2011-501740A

### NON-PATENT LITERATURE

Non Patent Literature 1: Adi Millman et al., Cell, 2020, 183(6), p.1551-1561

### SUMMARY OF INVENTION

### TECHNICAL PROBLEM

Therefore, an object of the present invention is to provide a recombinant phage which can inactivate an anti-phage defense system of a bacterium including an antimicrobial-resistant bacterium, infect the bacterium, proliferate in the bacterium, and finally kill the bacterium, and a method for preparing the recombinant phage.

### SOLUTION TO PROBLEM

As a result of intensive studies to solve the above problems, the present inventors have found a tRNA rich region (hereinafter, also abbreviated as TRR) in a T5-like phage, which is not found in a general phage. Then, the present inventors have found that the problems of the present invention can be solved by incorporating at least one gene encoded within the TRR into a phage by genetic recombination to construct a recombinant phage capable of producing a protein encoded by the gene, and have completed the present invention.

The present invention has the following configuration.
1. A recombinant phage including at least one of genes encoded within a tRNA rich region (TRR) of a T5-like phage.
2. The recombinant phage according to the above 1, in which the recombinant phage is modified so that at least one of the genes encoded within the TRR is enhanced.
3. The recombinant phage according to the above 1 or 2, in which at least one of the genes encoded within the TRR is contained in a phage genome.
4. The recombinant phage according to any one of the above 1 to 3, in which the gene encoded within the TRR is at least one of a gene encoding a Retron inhibitor and a gene encoding a tRNA functioning in a target bacterium.
5. The recombinant phage according to the above 4, in which the Retron inhibitor is a specific degrading enzyme for an ncRNA in the Retron.
6. The recombinant phage according to the above 4, in which the gene encoding the Retron inhibitor includes any one selected from nucleotide sequences represented by SEQ ID NO: 5 to SEQ ID NO: 9.
7. The recombinant phage according to the above 6, in which the Retron inhibitor includes any one selected from amino acid sequences represented by SEQ ID NO: 10 to SEQ ID NO: 14.
8. The recombinant phage according to any one of the above 4 to 7, in which the tRNA functioning in the target bacterium is a tRNA that inhibits an anti-phage defense system in the target bacterium.
9. The recombinant phage according to any one of the above 4 to 8, in which the tRNA functioning in the target bacterium is a tRNA to be degraded by an effector protein contained in the anti-phage defense system in the target bacterium.
10. The recombinant phage according to the above 9, in which the gene encoding the tRNA functioning in the target bacterium includes any one selected from nucleotide sequences represented by SEQ ID NO: 15 to SEQ ID NO: 19.
11. The recombinant phage according to any one of the above 1 to 10, which is a lytic phage.
12. An antibacterial agent or bacteriological testing agent containing the recombinant phage according to the above 11.
13. A composition containing the antibacterial agent or the bacteriological testing agent according to the above 12.
14. A sterilization method for killing a target bacterium, the method including bringing the composition according to the above 13 into contact with the target bacterium.
15. The sterilization method according to the above 14, in which the target bacterium is a pathogenic bacterium.
16. The sterilization method according to the above 15, in which the pathogenic bacterium is at least one selected from Campylobacter jejuni, Vibrio cholerae, Escherichia coli 0157, Legionella pneumophila, Yersinia pestis, Salmonella, and Shigella.
17. A method for preparing a recombinant phage, the method including the following steps (1) to (3):
   step (1) of preparing the following a) and b):
      a) a phage genome; and
      b) a nucleotide including at least one of genes encoded within a TRR;
   step (2) of obtaining a recombinant phage genome by inserting (b) the nucleotide onto (a) the phage genome;
   step (3) of obtaining a recombinant phage into which the recombinant phage genome has been introduced; and
   step (4) of collecting the recombinant phage.
18. The method for preparing a recombinant phage according to the above 17, in which the gene encoded within the TRR is at least one of a gene encoding a Retron inhibitor and a gene encoding a tRNA functioning in a target bacterium.
19. A recombinant phage including a gene encoding a protein that is an effector protein constituting the Retron in a bacterium and is a tRNA cleavage enzyme in the bacterium.
20. The recombinant phage according to the above 19, in which the gene encoding the protein is contained in a phage genome.
21. The recombinant phage according to the above 19 or 20, in which the gene encoding the effector protein includes at least one of nucleotide sequences represented by SEQ ID NO: 20 and SEQ ID NO: 21.

### ADVANTAGEOUS EFFECTS OF INVENTION

The recombinant phage of the present disclosure can inactivate an anti-phage defense system of a bacterium for defending phage infection. Specifically, by incorporating at least one of genes encoded within the TRR, which is an inhibitor of an anti-phage defense system, into a phage, the anti-phage defense system can be inactivated, and the phage can infect bacteria and exhibit resistance to the anti-phage defense system.

In addition, the recombinant phage of the present disclosure can kill drug-resistant bacteria because it has a sterilization mechanism different from that of an antibacterial drug according to the related art. The present invention can also be applied to various types of phages by using genetic recombination. A composition containing the recombinant phage of the present disclosure can be used as a bacteria agent. In addition, the recombinant phage of the present disclosure is harmless to the human body because it infects only bacteria. Therefore, the antibacterial agent of the present disclosure can be used not only by being applied to the skin or the like but also as a beverage.

### BRIEF DESCRIPTION OF DRAWINGS

[FIG. 1] FIG. 1 is a schematic diagram of a mechanism of inhibiting, in the case where Phage SP15 infects a bacterium having a Retron Ec78 anti-phage defense system, the Retron.
[FIG. 2] a of FIG. 2 is a schematic diagram in which genomes of phages are compared with one another. b and c of FIG. 2 are schematic diagrams showing a method of a spot assay. c of FIG. 2 shows an index of infectivity by the spot assay.
[FIG. 3] a of FIG. 3 shows results of the spot assay. b of FIG. 3 is a schematic diagram of a procedure for dividing an SP15 phage-derived DRR into nine fragments and preparing a recombinant plasmid into which the fragments were inserted. c of FIG. 3 is a schematic diagram of F6 and F8.
[FIG. 4] FIG. 4 shows the results of the spot assay.
[FIG. 5] a to c of FIG. 5 show results of a spot assay.
[FIG. 6] FIG. 6 shows results of evaluating an amount of msDNA after introducing a recombinant plasmid having a nucleotide encoding Rad into a bacterium having Retron.
[FIG. 7] a and b of FIG. 7 show results of a spot assay.
[FIG. 8] FIG. 8 shows results of a spot assay.
[FIG. 9] a of FIG. 9 is a simplified diagram of a method for evaluating an intracellular target of ptuAB of Retron Ec78. b of FIG. 9 shows results of a cytotoxicity test, and c and d of FIG. 9 show results of dot blot RNA hybridization.
[FIG. 10] FIG. 10 shows results of tRNA-sequencing.
[FIG. 11] a of FIG. 11 shows a structure of tRNA-Tyr, b of FIG. 11 shows a tRNA-Tyr phylogenetic tree, and c of FIG. 11 shows sequence alignments of various kinds of tRNA-Tyr.
[FIG. 12] a to d of FIG. 12 show results of a spot assay.
[FIG. 13] FIG. 13 is a schematic diagram showing an outline of phage genome sequencing.
[FIG. 14] FIG. 14 shows results of the phage genome sequencing.
[FIG. 15] a and b of FIG. 15 show results of a spot assay.
[FIG. 16] FIG. 16 shows results of a spot assay.
[FIG. 17] a to d of FIG. 17 show a sterilization test using a phage into which a Retron inhibitor (Rad) is incorporated. a of FIG. 17 is a schematic diagram showing construction of a T7 phage containing SP15-derived Rad using a synthesis platform. b of FIG. 17 is a schematic diagram in which the Rad is inserted into the T7 phage. c of FIG. 17 shows results of a spot assay. d of FIG. 17 shows results of a phage titer measurement.
[FIG. 18] FIG. 18 shows results of a spot assay using Escherichia coli harboring DRT-type 1 and DRT-type 3.
[FIG. 19] FIG. 19 shows an example of synthesis of a T7 phage into which tRNALys is incorporated.
[FIG. 20] FIG. 20 shows that tRNA also exhibits an inhibitory function in a PrrC defense system. a is a heat map showing an anti-phage function of PrrC170 anticodon nuclease. b is a diagram of proliferation termination observed in a bacterium expressing a PrrC toxin. c shows results of expression of tRNAs in bacteria harboring PrrC. d is a diagram showing a functional failure of a PrrC170 anti-phage defense system in bacteria expressing tRNA-Lys.

### DESCRIPTION OF EMBODIMENTS

In the present description, the notation may be abbreviated as follows.

The bacteriophage is also simply referred to as "phage".

### <tRNA Rich Region (TRR) in T5-Like Phage>

A recombinant phage of the present embodiment contains at least one of genes encoded within a tRNA rich region (TRR) of a T5-like phage. Examples of the gene encoded within the tRNA rich region (TRR) of the T5-like phage include a gene encoding a Retron inhibitor, a gene encoding a Septu inhibitor, a gene encoding tRNA functioning in a target bacterium, a gene encoding Avast, and a gene encoding upx. Among them, a gene encoding a Retron inhibitor, a gene encoding a Septu inhibitor, and a gene encoding a tRNA functioning in a target bacterium are preferred, and a gene encoding at least one of a Retron inhibitor and a Septu inhibitor, and a gene encoding a tRNA functioning in a target bacterium are more preferred.

### <Anti-Phage Defense System>

In the present description, an "anti-phage defense system" of bacteria refers to a generic term for a mechanism of protecting bacteria from phages (viruses infecting bacteria). When a phage infects a bacterium, the bacterium is lysed to release progeny phages, and surrounding bacteria are also sterilized to end the colony. When the target bacterium has an anti-phage defense system, even if the phage infects the bacterium, abortive infection is induced, the bacterium dies (self-destructs) before the phage matures, and the survival of the colony is maintained.

Examples of anti-phage defense systems that have been reported so far include Retron, Septu, CRISPR-Cas, restriction-modification enzymes, and Argonaute.

### <<Retron>>

Retrons is a bacterial gene element composed of a reverse transcriptase (hereinafter also abbreviated as RT) and a non-coding RNA (hereinafter also abbreviated as an ncRNA). The reverse transcriptase uses the ncRNA as a template to produce a chimeric RNA/DNA molecule in which an RNA and a DNA are covalently bonded. Retron functions as an anti-phage defense system and is composed of three elements, i.e., RT, ncRNA, and effector protein (Adi Millman et al., Cell, 2020, 183(6), p.1551-1561).

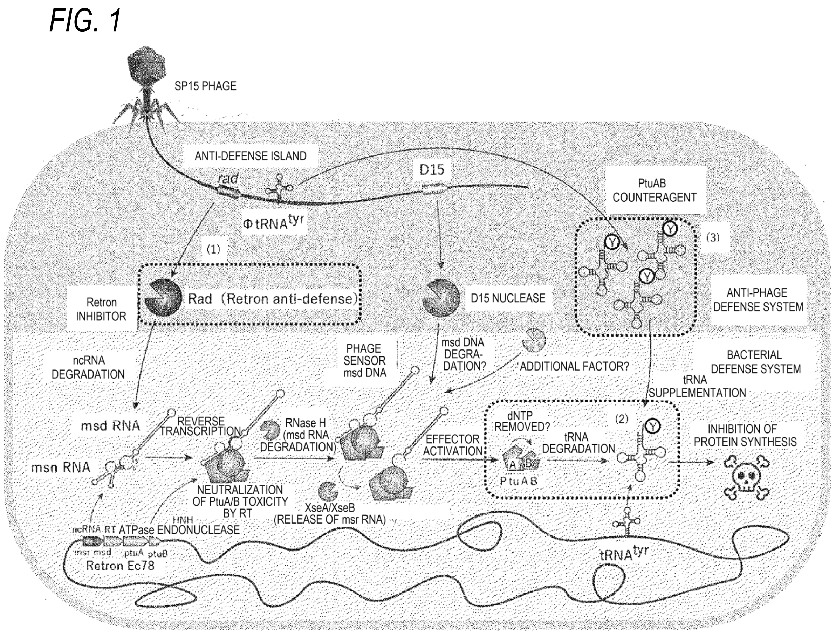

The present inventors have found the following [1] and [2] as a mechanism in which a phage inhibits a Retron anti-phage defense system. As a specific example, FIG. 1 is a schematic diagram of a mechanism of inhibiting, in the case where Phage SP15 infects a bacterium having a Retron Ec78 anti-phage defense system, the Retron.

The phage has three genetic factors including Rad that inhibits biosynthesis of Retron, a Retron trigger (D15 in the case of Ec78, and DenB or A1 in the case of Ec67), and a tRNA that supplies and complements a host tRNA degraded by Retron Ec78, and is related to Retron defense.

After infection with the phage, Retron can sense a phage protein prepared in advance and packed in a capsid together with a phage genome or a protein expressed during the initial production of phage particles. It is considered that the generation of such a sensor/trigger activates the Retron defense system by the degradation of Retron components (most of the Retron triggers are involved in the degradation of nucleotides) or the Retron sensing the degradation of the host genomic DNA.

When Retron is activated, the effector protein is released from a Retron complex. It is suggested that when dNTP is excessively used for phage construction and dNTP is depleted, ptuAB is activated to cleave the tRNA of the bacterium, and as a result, the growth of the bacterium is stopped to stop the production of phages [(2) in FIG. 1].

In order to evade the Retron anti-phage defense system, the recombinant phage of the present embodiment contains at least one of a gene encoding the following [1] Rad and a gene encoding [2] a tRNA.
[1] Rad that is a Retron inhibitor [(1) in FIG. 1].
   When a region encoding the ncRNA in the Retron is specifically degraded by Rad, the activation of the anti-phage defense system in the Retron is inhibited. In the present embodiment, it is preferable that Rad can also function as a Septu inhibitor.
[2] Replenishment of tRNAs destroyed by an effector protein in a bacterial anti-phage defense system [(2) and (3) in FIG. 1].

A DNA produced with a reverse transcriptase from an ncRNA in the Retron of the bacterium is cleaved by a phage-derived nuclease to activate an effector protein (PtuAB in FIG. 1). When the tRNA of the bacterium is degraded by the activated effector protein, protein synthesis is inhibited, and the bacterium undergoes self-destruction [(2) in FIG. 1]. The tRNA degraded by the anti-phage defense system is replenished with a phage to prevent self-destruction of the bacteria and establish infection [(3) in FIG. 1]. The tRNA replenished from the phage is shown as "PtuAB's counteragent" in FIG. 1.

Therefore, by incorporating at least one of [1] the Retron inhibitor and [2] the tRNA functioning in the target bacterium into the recombinant phage, infection with the phage for the bacterium having an anti-phage defense system based on the Retron can be established. Retron is applied to gene recording (Santi Bhattarai-Kline et al., Nature, 2022, 608, p.217-225), and it is considered that the mechanism of inhibiting the Retron can also be used to inhibit the reaction of gene recording.

That is, examples of an aspect of the present embodiment include the following:
- a recombinant phage containing a gene encoding [1] the Retron inhibitor;
- a recombinant phage containing a gene encoding [2] the tRNA functioning in a target bacterium; and
- a recombinant phage containing a gene encoding [1] the Retron inhibitor and a gene encoding [2] the tRNA functioning in a target bacterium.

### <<Septu>>

Septu is a system for controlling replication and transcription of a virus genome in a phage (a virus infecting bacteria) [Barrangou, R., et al., 2007, Science, 315(5819), 1709-1712]. Septu contains a CRISPR sequence, which is a part of a virus genome, and a Cas gene associated with the CRISPR sequence. The CRISPR sequence retains fragments from the virus genome and provides an immune response to a virus. The Cas gene recognizes the virus genome using the CRISPR sequence and encodes an enzyme that cleaves the virus genome. The Septu system plays a role of protecting bacteria infected with viruses by destroying the virus genome.

In one aspect of the present embodiment, at least one of the genes encoded within the TRR is preferably a gene encoding an inhibitor of at least one of Retron and Septu, and more preferably a gene encoding an inhibitor of Retron and Septu.

### <Retron Inhibitor>

Examples of the Retron inhibitor include a specific degrading enzyme for a region encoding a non-coding RNA (also abbreviated as ncRNA) in Retron of a target bacterium. Examples of the Retron inhibitor include Rad shown in Table 1.

### [Table 1]

**Table 1**

| SEQ ID NO: | Names | Sequence | Derivatives |
|---|---|---|---|
| 5 | Rad-SP15 | Nucleotide sequence | SP15 phage |
| 6 | Rad-T5j | Nucleotide sequence | T5j phage |
| 7 | Nucleotide of Rad-Salmonella vB Sen 11 | Nucleotide sequence | Salmonella vB Sen 11 phage |
| 8 | Nucleotide of Rad-Proteus phage Privateer | Nucleotide sequence | Proteus phage Privateer phage |
| 9 | Nucleotide of Rad-Shigella Sonnei phage | Nucleotide sequence | Shigella Sonnei phage phage |
| 10 | Amino acid of Rad-SP15 | Amino acid sequence | SP15 phage |
| 11 | Amino acid of Rad-T5j | Amino acid sequence | T5j phage |
| 12 | Amino acid of Rad-Salmonella vB Sen 11 | Amino acid sequence | Salmonella vB Sen 11 phage |
| 13 | Amino acid of Rad-Proteus phage Privateer | Amino acid sequence | Proteus phage Privateer phage |
| 14 | Amino acid of Rad-Shigella Sonnei phage | Amino acid sequence | Shigella Sonnei phage phage |

The Retron inhibitor specifically degrades the region encoding the ncRNA in the Retron anti-phage defense system to inhibit the activation of the Retron anti-phage defense system. Examples of the ncRNA include ncRNAs in Retron EC67, Retron EC78, or Retron EC83.

The Retron inhibitor allows mutant phages of a wild-type phage to infect bacteria having a Retron anti-phage defense system, and a mutant phage resistant to the bacteria is isolated. A gene encoding a Retron inhibitor in an anti-phage defense system can be isolated by comparing a genome of the mutant phage exhibiting the resistance with a genome of the wild-type phage. Whether the gene corresponds to the Retron inhibitor can be determined by infecting a bacterium having a Retron anti-phage defense system with a recombinant phage obtained by introducing a nucleotide containing the gene into a phage genome, and examining whether the infection can be established. It can also be determined by expressing the nucleotide containing the gene in a bacterium having an anti-phage defense system in advance with a plasmid or the like, infecting the bacterium with a phage genome that is deficient in the nucleotide containing the gene, and examining whether the infection can be established.

### <tRNA Functioning in Target Bacterium>

In the present embodiment, a tRNA functioning in the target bacterium is preferably a tRNA that inhibits the anti-phage defense system in the target bacterium. The tRNA functioning in the target bacterium is preferably a tRNA that is degraded by an effector protein contained in the anti-phage defense system in the target bacterium.

From this viewpoint, the target bacterium is preferably a bacterium having an anti-phage defense system containing an effector protein capable of degrading the tRNA in the target bacterium or inhibiting a function of the tRNA in the target bacterium. The self-destruction of the bacterium due to depletion of the bacteria-derived tRNA can be prevented, and infection can be established by replenishing the tRNA degraded and inhibited by the protein contained in the anti-phage defense system of the target bacterium with a phage.

Examples of the anti-phage defense system containing an effector protein capable of degrading the tRNA in the target bacterium include Retron, Septu, PrrC, AtaT, VapC, MazF, and HipA.

Examples of the effector protein capable of degrading a tRNA in a target bacterium include proteins shown in Table 2.

### [Table 2]

**Table 2**

| SEQ ID NO: | Names | Sequence | Derivatives |
|---|---|---|---|
| 20 | PtuA | Nucleotide sequence | Escherichia coli ECONIH5 |
| 21 | PtuB | Nucleotide sequence | Escherichia coli ECONIH5 |
| 22 | PtuA | Amino acid sequence | Escherichia coli ECONIH5 |
| 23 | PtuB | Amino acid sequence | Escherichia coli ECONIH5 |

Examples of the tRNA functioning in the target bacterium include tRNAs that are degraded by an effector protein in the anti-phage defense system of the target bacterium. Examples of the tRNA include tRNAs encoding at least one selected from tyrosine, cysteine, aspartic acid, asparagine, serine, glutamic acid, glutamine, proline, glycine, alanine, arginine, isoleucine, leucine, lysine, methionine, phenylalanine, threonine, tryptophan, valine, and histidine.

The tRNA incorporated into the recombinant phage is preferably a tRNA (endogenous) derived from a phage into which the tRNA is introduced or a phage (exogenous) that is genetically closely related to the phage. The length of the tRNA is not particularly limited, and is generally preferably 76 bp to 91 bp.

Examples of the gene encoding the tRNA functioning in the target bacterium include a gene containing any one selected from the nucleotide sequences shown by SEQ ID NO: 15 to SEQ ID NO: 19. The nucleotide sequences of the gene encoding the tRNA are shown in Table 3.

### [Table 3]

**Table 3**

| SEQ ID NO: | Names | Derivatives |
|---|---|---|
| 15 | tRNA-Tyr T5 | T5 phage |
| 16 | tRNA Tyr SP15 | SP15 phage |
| 17 | Ec tRNA-TyrU | Ec phage |
| 18 | Ec tRNA-TyrV | Ec phage |
| 19 | tRNA-Tyr VB KpP HS106 | Klebsiella phage VB KpP HS106 |

The tRNA functioning in the target bacterium allows mutant phages of a wild-type phage to infect bacteria having a Retron anti-phage defense system, and a mutant phage resistant to the bacteria is isolated. A gene encoding the tRNA functioning in the target bacterium having an anti-phage defense system can be isolated by comparing a genome of the mutant phage exhibiting the resistance with a genome of the wild-type phage. Whether the gene is a gene encoding the tRNA can be determined by infecting a bacterium having an anti-phage defense system with a recombinant phage obtained by introducing a nucleotide containing the gene into a phage genome, and examining whether the infection can be established. It can also be determined by expressing the nucleotide containing the gene in a bacterium having an anti-phage defense system in advance with a plasmid or the like, infecting the bacterium with a phage genome that is deficient in the nucleotide containing the gene, and examining whether the infection can be established.

### <<Target Bacterium>>

The target bacterium is not particularly limited, and there are no particular restrictions on the shape (generally classified into a spherical shape, a rod shape, and a helical shape), Gram stainability (Gram-positive or Gram-negative), oxygen requirement (aerobic, anaerobic, facultative anaerobic), the form of existence, or the like.

Examples of the target bacterium include Escherichia coli, Shigella (such as S.dysenteriae, S.frexneri, and S.sonnei), Salmonella (such as S.typh, S.paratyphi-A, S.schottmuelleri, S.typhimurium, and S.enteritidis), Enterobacter (such as E.aerogenes and E.cloacae), Klebsiella (such as K.pneumoniae and K.oxytoca), Proteus (such as P.mirabilis and P.vulgaris), Yersinia (such as Y.pestis and Y.enterocolitica), Vibrio (such as Vcholerae and Vparahaemolyticus), Haemophilus (such as H.influenzae, H.parainfluenzae, and H.ducreyi), Pseudomonas (such as P.aeruginosa, P.cepacia, and P.putida), Acinetobacter (such as A.calcoaceticus, A.baumannii, and A.lwoffii), Legionella (such as L.pneumophila), Bordetella (such as B.pertussis, B.parapertussis, and B.bronchiseptica), Brucella (such as B.melitensis, B.abortus, and B.suis), Francisella tularensis, Bacteroides (such as B.fragilis and B.melaninogenicus), Neisseria (such as N.gonorrhoeae and N.meningitidis), Staphylococcus (such as S.aureus, S.epidermidis, and S.saprophyticus), Streptococcus (such as S.pyogenes, S.agalactiae, S.viridans, and S.pneumoniae), Enterococcus (such as E.faecalis, E.faecium, and E.avium), Bacillus (such as B.subtilis, B.anthracis, and B.cereus), Clostridium (such as C.difficile, C.botulinum, C.perfringens, and C.tetani), Corynebacterium (such as C.diphtheriae), Mycobacterium (such as M.tuberculosis, M.bovis, M.leprae, M.avium, M.intracellulare, M.kansasii, and M.ulcerans), Mycoplasma), Borrelia (such as B.recurrentis and B.burgdoferi), Treponema palidum, Campylobacter (such as C.coli, C.jejuni, and C.fetus), Helicobacter (such as H.pylori and H.heilmannii), Rickettsia (such as R.prowazekil, R.mooseri, and R.tsutsugamushi), Chlamydia (such as C.trachomatis and C.psittaci), Listeria (such as L.monocytogenes).

The target bacterium is preferably a pathogenic bacterium. Examples of the pathogenic bacterium include Campylobacter jejuni, Vibrio cholerae, Escherichia coli 0157, Legionella pneumophila, Yersinia pestis, Salmonella, and Shigella.

### <Recombinant Phage>

The recombinant phage of the present embodiment contains at least one of genes encoded within the TRR. The gene encoded within the TRR may be endogenous or exogenous. The gene encoded within the TRR is preferably at least one of a gene encoding the Retron inhibitor and a gene encoding the tRNA functioning in the target bacterium.

Specifically, for example, by incorporating a gene encoding a Retron inhibitor into a recombinant phage, an anti-phage defense system based on Retron in a target bacterium can be inhibited, and infection of the recombinant phage to the target bacterium can be established.

In addition, by incorporating a gene encoding a tRNA functioning in a target bacterium into a phage, a tRNA which is degraded or whose function is inhibited by an effector protein constituting an anti-phage defense system of the target bacterium is replenished by the phage, so that self-destruction of the bacterium due to depletion of tRNAs derived from the target bacterium is prevented, and infection is facilitated.

Examples of a method for incorporating at least one of genes encoded within the TRR into a phage include a method of inserting a gene into a phage genome, a method of inserting a gene when assembling a phage genome in vitro or in a cell and rebooting the inserted gene in a bacterial cell or in a test tube, a method of recombining a phage gene in a bacterial cell, and a method of recombining a phage gene in a bacterial cell (Diana P. Pires et al., Microbiology and Molecular Biology Reviews, 2016, Vol. 80, No. 3).

Specifically, for example, in a phage genome, as a site into which a gene encoding the Retron inhibitor is inserted, for example, a region that controls metabolism in an early stage of infection, a region that controls DNA amplification of a phage, or a capsid synthesis region having a high expression level is preferable.

For example, in a phage genome, as a site into which a gene sequence encoding a tRNA is inserted, for example, a region that controls metabolism at an early stage of infection, a region that controls DNA amplification of a phage, or a capsid synthesis region having a high expression level is preferable.

The recombinant phage of the present embodiment is preferably a recombinant phage modified so that the expression of at least one of genes encoded within the TRR is enhanced. The gene encoded within the TRR is preferably at least one of a gene encoding the Retron inhibitor and a gene encoding the tRNA functioning in the target bacterium.

Examples of a method for enhancing the expression of the gene include causing multiple copies of the gene to be present on a phage genome, replacing an expression regulatory sequence, such as a promoter of the gene, with a strong one, optimizing a codon, and enhancing enzyme activity.

Examples of the promoter include a rRNA promoter, a housekeeping gene promoter, and a LexA promoter.

### <<Phage>>

In the present embodiment, examples of the phage include lytic phages and temperate phages, and lytic phages are preferable. The lytic phage infects a host bacterium and proliferates inside the bacterium, then lyses the bacterium from the inside, and the proliferated phage is released to the outside of the bacterium. At this time, the bacteria are killed. Unlike the temperate phage, the phage itself is proliferated in the bacterium without going through the lytic cycle, and thus there is no risk of incorporating the genomic DNA of the host bacterium and acquiring the toxicity of the host cell, and the phage does not become a part of the genome of the host. Accordingly, the lytic phage is preferable from the viewpoint of safety.

Examples of lytic phages include phages of Myoviridae phages (genus T4-like viruses, genus P1-like viruses, genus P2-like viruses, genus Mu-like viruses, genus SPO1-like viruses, and genus phiH-like viruses), Siphoviridae phages (genus λ-like viruses, genus γ-like viruses, genus Tl-like viruses, genus T5-like viruses, genus c2-like viruses, genus L5-like viruses, genus PsiM1-like viruses, genus phiC31-like viruses, and genus N15-like viruses), Podoviridae phages (genus T7-like viruses, genus phi29-like viruses, genus P22-like viruses, and genus N4-like viruses), Tectiviridae phages (genus Tectivirus), Corticoviridae phages (genus Corticovirus), Lipothrixviridae phages (genus Alphalipothrixvirus, genus Betalipothrixvirus, genus Gammalipothrixvirus, and genus Deltalipothrixvirus), Plasmaviridae phages (genus Plasmavirus), Rudiviridae phages (genus Rudivirus), Fuselloviridae phages (genus Fusellovirus), Inoviridae phages (genus Inovirus, genus Plectrovirus, genus M13-like virus, and genus fd-like virus), Microviridae phages (genus Microvirus, genus Spiromicrovirus, genus Bdellomicrovirus, and genus Chlamydiamicrovirus), Leviviridae phages (genus Levivirus, and genus Allolevivirus), Cystoviridae phages (genus Cystovirus), Ampullaviridae phages, Bicaudaviridae phages, Clavaviridae phages, Globuloviridae phages, and Guttavirus phages.

Examples of the temperate phage include, but are not particularly limited to, the Phietavirus family, the Lambdavirus family, and the Punavirus family.

### <Method for Preparing Recombinant Phage>

The recombinant phage of the present embodiment can be prepared according to a method including the following steps (1) to (4):
step (1) of preparing the following a) and b):
   a) a phage genome; and
   b) a nucleotide containing at least one of genes encoded within the TRR;
step (2) of obtaining a recombinant phage genome by inserting (b) the nucleotide onto (a) the phage genome;
step (3) of obtaining a recombinant phage into which the recombinant phage genome has been introduced; and
step (4) of collecting the recombinant phage.

Hereinafter, each step will be described.

### <<Step (1)>>

Step (1) is a step of preparing a nucleotide containing a) the phage genome and b) at least one of genes encoded within the TRR.

In a), examples of the phage genome include a genome derived from the phage described above in the section <<Phage>>.

In b), the gene encoded within the TRR is preferably at least one of a gene encoding a Retron inhibitor and a gene encoding a tRNA functioning in a target bacterium.

Examples of the nucleotide containing a gene encoding a Retron inhibitor include a nucleotide containing any one selected from the nucleotide sequences represented by SEQ ID NO: 5 to SEQ ID NO: 9.

Examples of the nucleotide containing a gene encoding a tRNA functioning in a target bacterium include a nucleotide containing any one selected from nucleotides represented by SEQ ID NO: 15 to SEQ ID NO: 19.

### <<Step (2)>>

Step (2) is a step of obtaining a recombinant phage genome by inserting b) the nucleotide onto a) the phage genome prepared in step (1).

Examples of the method of inserting b) the nucleotide onto a) the phage genome include the methods described above in the section <Recombinant Phage>.

### <<Step (3)>>

Step (3) is a step of obtaining a recombinant phage into which the recombinant phage genome obtained in step (2) has been introduced. The recombinant phage is obtained by introducing the recombinant phage genome obtained in step (2) into a bacterium to obtain a phage-synthesizing bacterium, and causing the phage-synthesizing bacterium to produce the recombinant phage.

Examples of a method of introducing a phage genome into a bacterium include a competent cell method and an electroporation method, and a phage genome is introduced into a bacterium according to a common method. Examples of the bacterium into which the recombinant phage genome is to be introduced include common bacteria such as Escherichia coli.

### <<Step (4)>>

Step (4) is a step of collecting a recombinant phage from the phage-synthesizing bacterium obtained in step (3). Specifically, the recombinant phage is collected by lysing the recombinant phage from the phage-synthesizing bacterium or releasing the recombinant phage to the outside of the bacterial cells. As a method of collecting the recombinant phage, a known method can be used.

### <Effector Protein in Retron>

Examples of an embodiment of the present embodiment includes a recombinant phage containing a gene encoding a protein that is an effector protein constituting the Retron in a bacterium and is a tRNA cleavage enzyme in the bacterium.

The effector protein in the Retron is preferably a tRNA cleavage enzyme in a target bacterium. Examples of the effector protein in the Retron, which is a tRNA cleavage enzyme in a target bacterium, include PtuA (amino acid sequence: SEQ ID NO: 20, nucleotide sequence: SEQ ID NO: 22) and PtuB (amino acid sequence: SEQ ID NO: 21, nucleotide sequence: SEQ ID NO: 23).

The cell proliferation of the target bacterium can be prevented by incorporating a gene encoding the effector protein into a recombinant phage. Examples of a method of incorporating the gene encoding the effector protein into a recombinant phage include a method of inserting the gene onto a phage genome, a method of inserting the gene when assembling a phage genome in vitro or in a cell, and rebooting the gene in the bacterial cell or in vitro, and a method of recombining phage genes in the bacterial cell.

### <Antibacterial Agent or Bacteriological Testing Agent and Composition Containing Antibacterial Agent or Bacteriological Testing Agent>

The recombinant phage according to the present embodiment is useful as an active ingredient of an antibacterial agent because it exhibits excellent infectivity to a bacterium having an anti-phage defense system. The "antibacterial agent" refers to an agent having an action/effect of inhibiting the proliferation of bacteria (bacteriostasis) or an action/effect of killing bacteria (sterilization). The antibacterial agent or the bacterial testing agent of the present embodiment is used in various applications as a composition containing the antibacterial agent or the bacteriological testing agent. The antibacterial agent or the bacteriological testing agent and the composition according to the present embodiment may be in a dried state or in a state of being dissolved in a solution.

The concentration of the recombinant phage in the antibacterial agent of the present embodiment can be appropriately selected and changed according to various conditions such as target bacteria and applications, and is generally preferably 10⁶ PFU/ml to 10¹⁰ PFU/ml, and more preferably 10⁸ PFU/ml to 10¹⁰ PFU/ml.

Hereinafter, as representative applications of the composition of the present embodiment, a pharmaceutical (for treatment, prevention, or bacterial testing) composition, a disinfecting composition, a cleaning composition, and an agricultural chemical composition using the composition according to the present embodiment will be described.

### (i) Pharmaceutical Composition

The pharmaceutical composition according to the present embodiment is used for treatment, prevention, or bacterial testing (phage typing) of bacterial infection. The pharmaceutical composition according to the present embodiment can exhibit a therapeutic effect or a preventive effect on bacterial infection. The therapeutic effect or the preventive effect here includes (1) inhibition of bacterial infection, (2) prevention, inhibition, or delay of the onset of bacterial infection, (3) alleviation (mildness) of symptoms characteristic of bacterial infection or accompanying symptoms, (4) prevention, inhibition, or delay of deterioration in symptoms characteristic of bacterial infection or accompanying symptoms, and the like. The therapeutic effect and the preventive effect are concepts that partially overlap each other.

The pharmaceutical composition can be formulated according to a common method. The pharmaceutical composition according to the present embodiment may contain an appropriate pharmaceutically acceptable carrier in order to prepare a pharmaceutically acceptable excipient. The carrier may contain a biocompatible material such as silicone, collagen, or gelatin. Alternatively, various emulsions may be used. Further, one or more formulation additives selected from, for example, diluents, fragrances, preservatives, excipients, disintegrants, lubricants, binders, emulsifiers, and plasticizers may be contained.

The dosage form for formulation is not particularly limited. Examples of the dosage form include tablets, powders, fine granules, granules, capsules, syrups, injections, external preparations (ointments, creams, lotions, liquids, gels, poultices, plasters, tapes, aerosols, etc.), and suppositories. One aspect of the pharmaceutical composition according to the present embodiment can be prescribed using a pharmaceutically acceptable excipient well known in the art in an administration form suitable for administration for oral administration.

The route of administration of the pharmaceutical composition according to the present embodiment is not particularly limited, and oral administration or parenteral administration can be performed. As the parenteral administration, for example, intravenous administration, intra-arterial administration, subcutaneous administration, intradermal administration, intramuscular administration, intraperitoneal administration, or direct administration to bacterial flora or infected sites can be performed. This administration method may be performed in the same manner as in phage therapies according to the related art.

In addition, in order to use the pharmaceutical composition according to the present embodiment for the treatment described above, the administration interval and the dose can be appropriately selected and changed depending on various conditions such as a state of a disease and a state of a subject.

The dose per administration and the number of administrations of the pharmaceutical composition according to the present embodiment can be appropriately selected and changed depending on the purpose of administration and various conditions such as the age, body weight, and symptoms of a patient, and severity of a disease.

The number of times of administration and the period of administration may be only one, or may be one to several times daily, for several weeks, or the state of the disease is monitored, and the pharmaceutical composition may be administered again or repeatedly. The concentration of the recombinant phage in the pharmaceutical composition according to the present embodiment can be appropriately selected and changed depending on various conditions such as the condition of the disease and the state of the subject, and is generally preferably 10⁶ PFU/ml to 10¹⁰ PFU/ml, and more preferably 10⁸ PFU/ml to 10¹⁰ PFU/ml.

The pharmaceutical composition according to the present embodiment can also be used in combination with other pharmaceutical compositions and the like. The pharmaceutical composition according to the present embodiment may be administered simultaneously with other pharmaceutical compositions, or may be administered at intervals. The order of administration is not particularly limited.

In addition, in the pharmaceutical composition according to the present embodiment, the period during which the disease is ameliorated or alleviated is not particularly limited, and the disease may be ameliorated or alleviated temporarily, or may be ameliorated or alleviated for a certain period.

In addition, the pharmaceutical composition according to the present embodiment can be used to treat an organism, a part of a body of an organism, or a part thereof that has been extracted or excreted from an organism. The organism is not particularly limited, and may be, for example, an animal, a plant, or a fungus. Examples of the animal include humans, domestic animal species, and wild animals. Therefore, the pharmaceutical composition according to the present embodiment can also be used for animal treatment for treating animals. That is, the recombinant phage according to the present embodiment can also be used in an animal treatment method for various animals other than humans.

### (ii) Disinfecting Composition and Cleaning Composition

The disinfecting composition or the cleaning composition according to the present embodiment is used for, for example, disinfection or cleaning of a room (including a hospital room), a cooking room, a toilet, a washroom, a bathroom, and the like, disinfection or cleaning of tableware, cutlery (for example, a knife, a fork, and a spoon), cooking utensils (for example, a kitchen knife, a knife, a pot, a mixer, a microwave oven, and an oven), medical utensils and devices, and disinfection or washing of hands, fingertips, oral cavity, and the like. The disinfectant or cleaning agent of the present embodiment is, for example, in a liquid form (for example, a spray agent, and a lotion), a gel form, or a solid form (for example, powder), and is applied by coating, spraying, scattering, or the like. The disinfecting composition or the sterilizing composition of the present embodiment may be supported by or attached to a carrier (for example, in a sheet form) made of natural fibers, synthetic fibers, or the like, and may be used as a product used for wiping or the like, a mask for preventing infection, or the like.

Antibacterial or disinfecting components such as benzalkonium chloride, cetylpyridinium chloride, phenoxyethanol, isopropylmethylphenol, and chlorhexidine gluconate, a pH adjuster, a surfactant, an adsorbent, a carrier, and the like may be added to the disinfecting composition or the cleaning composition of the present embodiment. The concentration of the recombinant phage in the disinfecting composition and the cleaning composition according to the present embodiment can be appropriately selected and changed according to various conditions such as the condition of the disease and the state of the subject, and is generally preferably 10⁶ PFU/ml to 10¹⁰ PFU/ml, and more preferably 10⁸ PFU/ml to 10¹⁰ PFU/ml.

### (iii) Agricultural Chemical Composition

The agricultural chemical composition of the present embodiment effectively acts on various crops widely cultivated in agriculture and horticulture. For example, the agricultural chemical composition of the present embodiment is effective for cultivated monocotyledonous crops such as rice, wheat, and corn, and cultivated dicotyledonous plants such as kidney beans, soybeans, tomatoes, potatoes, eggplants, and cucumbers.

The agricultural chemical composition of the present embodiment is generally used by being mixed with various carriers in a liquid form, a solid form, a gas form, and the like. The agricultural chemical composition of the present embodiment may further contain a surfactant or other agrochemical adjuvants as necessary.

Examples of the form of the formulation include an emulsion, a wettable powder, a dry flowable, a flowable, a water soluble powder/granule, a granule, a fine granule, a granule, a powder, a coating agent, a spray formulation, an aerosol formulation, a microcapsule formulation, a fumigant formulation, and a smoke insecticide.

The concentration of the recombinant phage in the agrochemical composition according to the present embodiment can be appropriately selected and changed according to various conditions such as the situation of the subject, and is generally preferably 10⁶ PFU/ml to 10¹⁰ PFU/ml, and more preferably 10⁸ PFU/ml to 10¹⁰ PFU/ml.

### <Sterilization Method>

As a method for killing the target bacteria of the present embodiment, the antibacterial agent or composition of the present embodiment may be brought into contact with the target bacteria, and when the target bacteria are in a state of forming a colony on a solid medium or the like, the antibacterial agent or composition in a powder form or a solution form may be sprayed on the colony. In addition, when the target bacteria are present in the solution, the amount of the powder or the concentration of the liquid may be adjusted and added so as to obtain a necessary concentration. Examples of the target bacteria include the bacteria described above in the section <<Target Bacteria>>.

### Examples

Hereinafter, the present invention will be specifically described using Examples and Comparative Examples, but the present invention is not limited thereto.

### [Test Material and Method]

### (Medium and Buffer)

All the experiments were conducted using Luria Bertani (LB) broth (10 g of polypeptone, 10 g of sodium chloride, and 5 g of yeast extract per liter) or LB agar (obtained by adding 1.5% agar to LB). Phosphate buffered saline (PBS) (8.0 g NaCl, 0.2 g KCl, 1.44 g Na₂HPO₄, 0.24 g KH₂PO₄) was used for dilution of a bacterial solution, and a sodium magnesium (SM) buffer (5.8 g NaCl, 0.2 g MgSO•H₂O 50 ml 1M Tris-HCl (pH. 7.5), 5 mL 2% (w/v) Gelatin solution) was used for storage of the phage and dilution of the phage solution.

### (Bacterial Strains and Phages)

In Examples, the anti-phage defense system was expressed by Escherichia coli DH10B. The bacteria were shaken at 37°C and 200 rpm and cultured on the LB, and appropriate antibiotics were added thereto, unless otherwise specified. For the gene expressed in Escherichia coli, Tetracycline (10 µg/ml) or Chloramphenicol (20 µg/ml) was used to reliably maintain the plasmid.

The purified phage was proliferated by mixing 1% of the overnight culture of 0157 with the liquid LB, followed by incubating at 37°C overnight. Before the phage was concentrated according to the polyethylene glycol 6000-NaCl (PEG-NaCl) method, the host cells were removed by centrifugation (5000g, 10 minutes, 4°C) and filtered through a 0.2 µm filter.

### (Construction of Plasmid and Strain)

Retron Ec83, Ec48, and Se72 were synthesized by the Genewitz Corporation and cloned. For co-expression of retron and DRR fragments, retron Ec78 and Ec83 were cloned under their respective native promoters using plasmids having pSC101 origin of replication (ori). The DRR fragment of retron was constructed using pKLC235 as a backbone and using a pBAD-inducible promoter and pA15 ori. The DRR fragment of Retron Ec67 was constructed using pKLC835 as a backbone and using a pBAD-inducible promoter. The mutation of tRNATyr was prepared by inverse PCR. The plasmid was assembled using Hifi NEBuilder (NEB). The Escherichia coli DH10B was transformed with the plasmid construct using a heat shock method.

### (Bacteriophage Spot Assay)

The spot assay was performed according to the following procedure. A fresh culture medium was prepared by inoculating 10% of the product obtained by culturing overnight in LB supplemented with an antibiotic. The bacterial for the spot assay were prepared by mixing 100 µl of a fresh culture with 4 ml of LB top agar (LB, 0.5% agarose gel, 1 mM CaCl₂) and pouring the mixture into an LB plate. The phage was diluted with a magnesium sodium (SM) buffer and placed in a 96-well plate. To bacterial lawn sat (bacteria grown in a turf shape) were dropwise added 3 µl of phage solutions having different dilution ratios for 15 minutes or longer, respectively, followed by incubating at 37°C overnight.

The spot assay was performed according to the following procedure. To 4 ml of LB top agar (LB 0.5% agarose supplemented with 1 mM CaCl₂) were added 100 µL of bacterial cells cultured overnight, followed by pouring into an LB plate and drying for 10 minutes. Next, 3 µL of a phage solution diluted 10-fold was added dropwise to a plate using eight multi-channel pipettes, followed by being left, and then, the mixture was held at room temperature until drying and incubated at 37°C overnight. After overnight culture, formation of an inhibition circle (plaque) was recorded.

### (Structure Prediction and Alignment of Phage Protein)

The structure of the Rad protein was predicted by default parameters using AlphaFold211 in ColabFold12. Visualization of the predicted structure and the position of the amino acid mutation was performed using PyMOL program v.2.5.1. The protein domains were searched for by the HHpred server using pfam, COG_KOG, and PHROGs as target databases.

### (Sequence Alignment of PtuAB)

Protein sequences of PtuAB of retron Ec78, retron Ec83, retron Vp96, and retron Vc95 were aligned with one another by default parameters using BLASTp on the NCBI BLAST web interface 13.

### [Test Example 1] Identification of Retron Inhibitor Rad and tRNA Functioning in Target Bacterium

The ability to infect bacteria having different types of anti-phage defense systems was evaluated using four types of phages, T5j phage, SP15 phage, and T5n phage and SP15m phage which are deletion mutants thereof.

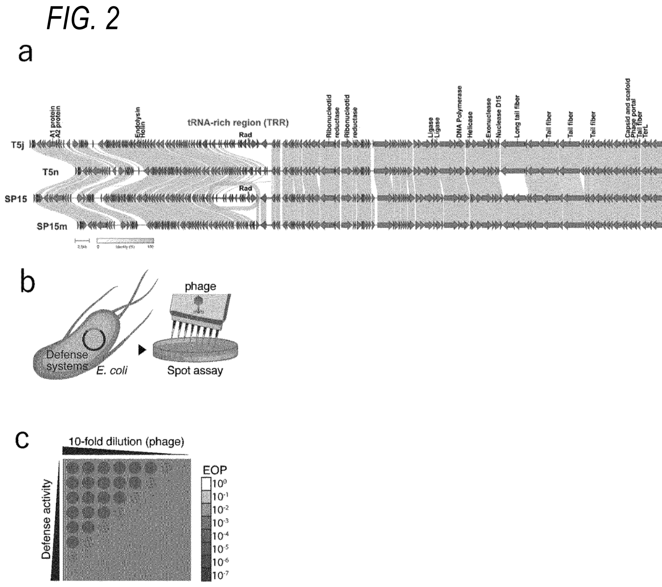

A nucleotide sequence of a genome of each phage is shown in Table 4. a of FIG. 2 is a schematic diagram in which the genomes of the phages are compared with each other. A genome region of about 8 kb (hereinafter, referred to as DRR) in T5j and SP15 is deficient in T5n and SP15m, respectively. Visualized genome comparison was made using Easyfig 1. As shown in a of FIG. 2, it was found that there was a large deletion (Defense Related Region, hereinafter referred to as DRR) in the genome.

### [Table 4]

**Table 4**

| SEQ ID NO: | Gene name | Derivatives |
|---|---|---|
| 1 | Genome of T5n | T5n phage |
| 2 | Genome of T5j | T5j phage |
| 3 | Genome of SP15m | SP15m phage |
| 4 | Genome of SP15 | SP15 phage |

Various bacteria each having an anti-phage defense system were infected with the phage, and whether the phage had infectivity was examined by spot assay. b of FIG. 2 is a schematic diagram illustrating a method of the spot assay, and c of FIG. 2 shows the index of the infectivity (EOP) by the spot assay.

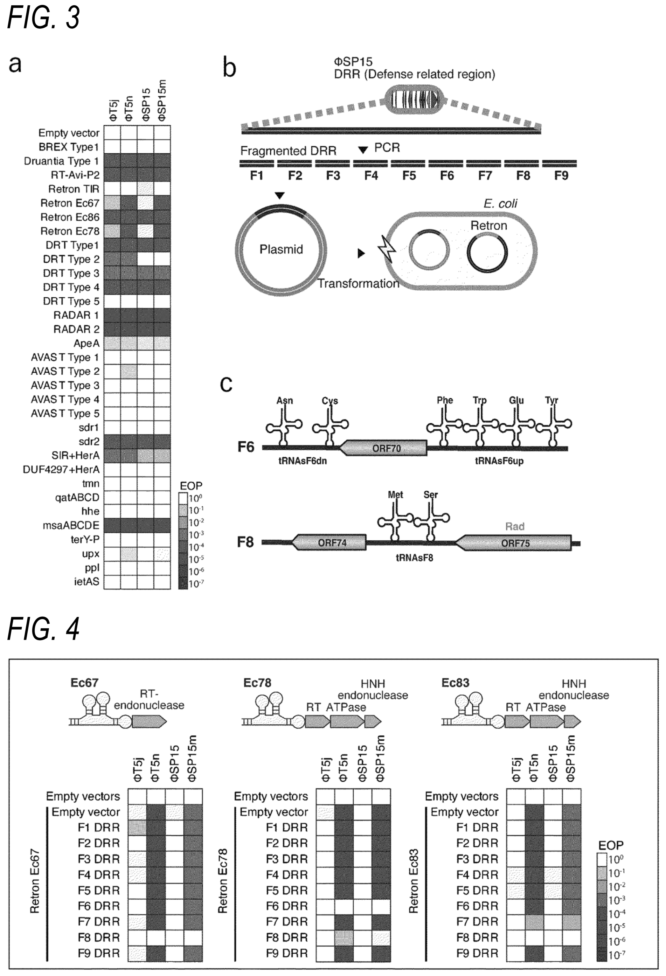

The results of the spot assay are shown in a of FIG. 3. As shown in a of FIG. 3, the infectivity of the deletion mutants T5n and SP15m phages to bacteria having Retron Ec67 and Retron Ec78 was significantly reduced. Therefore, as shown in b of FIG. 3, a DRR derived from the SP15 phage was divided into nine fragments, and each fragment was amplified by PCR and introduced into a plasmid pKLC23 having a pBAD-inducible promoter to prepare a recombinant plasmid. c of FIG. 3 is a schematic diagram of F6 and F8. The sequences of primers used in PCR are shown in Table 5.

### [Table 5]

**Table 5**

| SEQ ID NO: | Names | Purpose of amplification or PCR product | Template DNA |
|---|---|---|---|
| 24 | forward primer DRR1 | Insertion of F1 DRR | SP15 genome |
| 25 | reverse primer DRR1 | | SP15 genome |
| 26 | forward primer DRR2 | Insertion of F2 DRR | SP15 genome |
| 27 | reverse primer DRR2 | | SP15 genome |
| 28 | forward primer DRR3 | Insertion of F3 DRR | SP15 genome |
| 29 | reverse primer DRR3 | | SP15 genome |
| 30 | forward primer DRR4 | Insertion of F4 DRR | SP15 genome |
| 31 | reverse primer DRR4 | | SP15 genome |
| 32 | forward primer DRR5 | Insertion of F5 DRR | SP15 genome |
| 33 | reverse primer DRR5 | | SP15 genome |
| 34 | forward primer DRR6 | Insertion of F6 DRR | SP15 genome |
| 35 | reverse primer DRR6 | | SP15 genome |
| 36 | forward primer DRR7 | Insertion of F7 DRR | SP15 genome |
| 37 | reverse primer DRR7 | | SP15 genome |
| 38 | forward primer DRR8 | Insertion of F8 DRR | SP15 genome |
| 39 | reverse primer DRR8 | | SP15 genome |
| 40 | forward primer DRR9 | Insertion of F9 DRR | SP15 genome |
| 41 | reverse primer DRR9 | | SP15 genome |
| 42 | forward primer PDRR1 | pAZDRR1- | plasmid pKLC23 DNA |
| 43 | reverse primer PDRR1 | | plasmid pKLC23 DNA |
| 44 | forward primer PDRR2 | pAZDRR2- | plasmid pKLC23 DNA |
| 45 | reverse primer PDRR2 | | plasmid pKLC23 DNA |
| 46 | forward primer PDRR3 | pAZDRR3- | plasmid pKLC23 DNA |
| 47 | reverse primer PDRR3 | | plasmid pKLC23 DNA |
| 48 | forward primer PDRR4 | pAZDRR4- | plasmid pKLC23 DNA |
| 49 | reverse primer PDRR4 | | plasmid pKLC23 DNA |
| 50 | forward primer PDRR5 | pAZDRR5- | plasmid pKLC23 DNA |
| 51 | reverse primer PDRR5 | | plasmid pKLC23 DNA |
| 52 | forward primer PDRR6 | pAZDRR6- | plasmid pKLC23 DNA |
| 53 | reverse primer PDRR6 | | plasmid pKLC23 DNA |
| 54 | forward primer PDRR7 | pAZDRR7- | plasmid pKLC23 DNA |
| 55 | reverse primer PDRR7 | | plasmid pKLC23 DNA |
| 56 | forward primer PDRR8 | pAZDRR8- | plasmid pKLC23 DNA |
| 57 | rreverse primer PDRR8 | | plasmid pKLC23 DNA |
| 58 | forward primer PDRR9 | pAZDRR9- | plasmid pKLC23 DNA |
| 59 | reverse primer PDRR9 | | plasmid pKLC23 DNA |
| 60 | forward primer ORF75 | ORF75 | SP15 genome |
| 61 | reverse primer ORF75 | ORF75 | SP15 genome |
| 62 | forward primer pKL-75 | pAZ-75 | pKLC23 |
| 63 | reverse primer pKL-75 | pAZ-75 | pKLC23 |

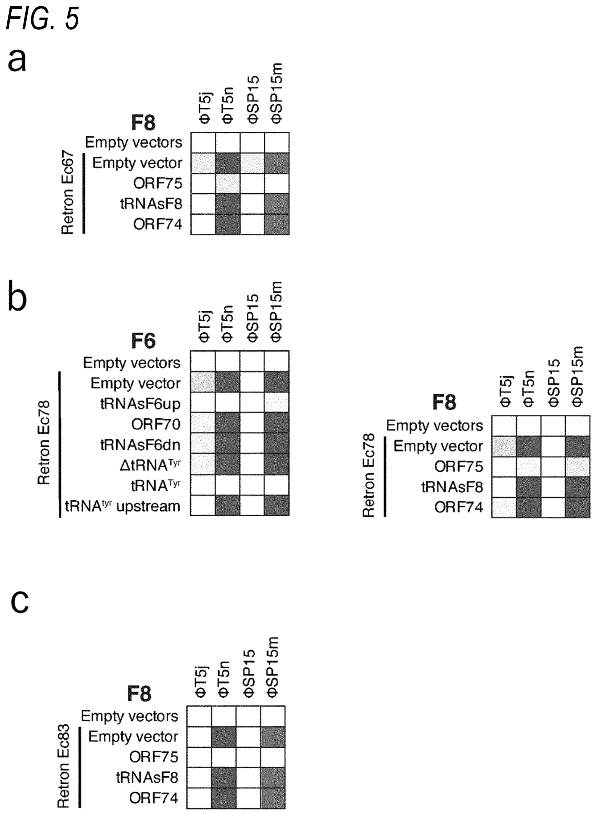

Escherichia coli expressing Retron Ec67, Retron Ec78, or Retron EC83 was transformed with the recombinant plasmid, and then the Escherichia coli was infected with each phage to examine whether the phage could be relieved from Retron. The results are shown in FIG. 4 and a to c of FIG. 5.

As a result, as shown in FIG. 4, the F6 DRR and the F8 DRR of the SP15 phage enabled the phage to evade three Retrons (Ec67, Ec78, and Ec83). As shown in a to c of FIG. 5, ORF75 (Rad) (nucleotide sequence: SEQ ID NO: 5, amino acid sequence: SEQ ID NO: 10) of the SP15 phage is a gene determining factor of F8 DRR, and the phage was enabled to evade three Retrons (Ec67, Ec78, and Ec83). Further, as shown in FIG. 4 and b of FIG. 5, tRNATyr (SEQ ID NO: 15) of F6 DRR enabled the phage to evade the Retron Ec78.

### [Test Example 2] Inhibition of Retron by Retron Inhibitor Rad

### (1) Inhibition of msDNA of Retron by Rad

The mechanism of inhibiting the Retron by the Retron Inhibitor Rad was analyzed. A recombinant plasmid having a nucleotide encoding SP15 phage-derived Rad was prepared. The recombinant plasmid was separately introduced into E. Coli having Retron Ec67, E. Coli having Retron Ec78, or E. Coli having Retron Ec83. After transformation, the Retron multi-copy single-stranded DNA (msDNA) was isolated, and the amount of msDNA was evaluated by electrophoresis using polyacrylamide gel.

Isolation of msDNA and evaluation based on the electrophoresis were performed according to the following procedure. Although ncRNA is highly expressed, it is necessary to overexpress ncRNA of the same type as RT in order to detect msDNA from Escherichia coli. Therefore, plasmids pEC67bad, pEC78bad, and pEC83bad, which encode RTs and ncRNAs of Retron Ec67, Retron Ec78, and Retron Ec83, respectively, were designed under the control of an inductive pBad promoter of pKLC231. The same plasmid without inserts, pKLC23, or those encoding RFPs instead of retron were used as negative controls. The plasmid pKLC83-R ad was designed to encode Rad under an anhydrous tetracycline (ATc)-inducible promoter, pKLC831. As a negative control, empty vector pKLC83 was used.

Escherichia coli DH10B cells containing plasmids pEC67, pEC78, and pEC83, or a negative control and pKL83-Rad, or a negative control were diluted by 1:100 with 25 mL LB medium supplemented with antibiotics (20 µg/ml chloramphenicol and 100 µg/ml ampicillin). After proliferation for 30 minutes with shaking at 37°C and 250 rpm, expression of retron was induced with 0.2% arabinose (final concentration), and proliferation was continued until OD600 reached 0.7. The samples were centrifuged at 4°C and 4000 rpm for 10 minutes, and then, the supernatants were discarded.

To isolate msDNA, each pellet was treated with three solutions. The three solutions are a solution I (50 mM glucose, 10 mM EDTA, 25 mM tris-hydrochloric acid, and pH 8.0), a solution II (0.2N NaOH and 1% SDS), and a solution III (3M potassium acetate and 2M acetic acid). The solution I was added to each pellet in 200 µL and vortexed. Next, 200 µL of the solution II was added, and each tube was inverted five times to perform mixing. Thereafter, 400 µL of the solution III was added, and the tube was inverted five times again to mix the samples. The samples were centrifuged at 4000 rpm and 4°C for 10 minutes, and then, the supernatants were collected.

Thereafter, the nucleic acid was precipitated using ethanol. To 700 µL of the collected supernatant was added 2.1 mL of 100% ethanol, followed by precipitation. The samples were centrifuged at 4°C and 13,000 rpm for 30 minutes, and the supernatants were discarded. In order to wash the pellets, 2.1 mL of 70% ethanol was added so as not to interfere with the pellets, and the samples were centrifuged at 4°C and 13,000 rpm for 10 minutes. The supernatants were discarded, and the samples were dried for 20 minutes and then resuspended in 20 µL of water.

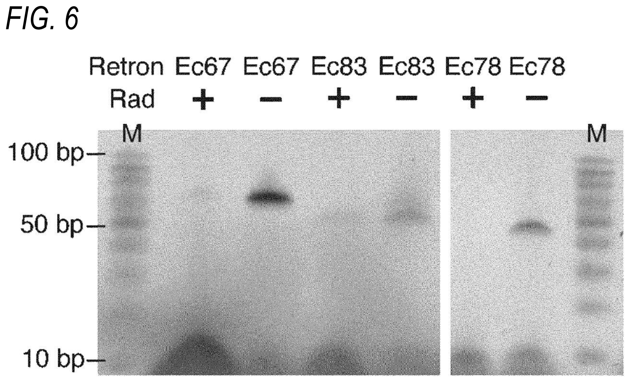

Thereafter, RNase A treatment was performed. To each sample, 2 µl of 10 µg/µl of RNase A (TAKARA (Japan)) was added. The sample was incubated at 37°C for 20 minutes. In order to facilitate visualization on the gel, a short DNA ladder (TAKARA, Japan) or a msDNA sample was diluted to 1:10. Next, 10 µl of each of these samples was mixed with a 2× TBE-urea loading dye and incubated at 70°C for 3 minutes. After cooling for 5 minutes, the sample was loaded into a 10% modified polyacrylamide gel and run at 180 V for 1 hour and 30 minutes. For visualization, the gel was washed once with TBE and then incubated on a SYBR gold bath for 40 minutes to perform staining. The results are shown in FIG. 6.

As shown in FIG. 6, it was found that the msDNA production of Retron was significantly reduced when co-expressed with Rad. From this result, it was found that Rad inhibits DNA synthesis in the Retron anti-phage defense system, and the amount of nucleic acid derived from Retron decreases. In addition, as a result of analysis by real-time PCR, it was found that Rad expression degrades Retron ncRNA.

### (2) Inhibition of Retron by Rad Homologue

Rad is a small protein with unknown function (189 amino acids), and has a primase/helicase domain and a TOPIRM/RNase domain. With the search based on homology, 541 Rad homologues were found in the 19,263 phage genomes in the database. The phage encoding Rad belongs to two phage families including the family Siphoviridae and the family Myoviridae, and infects at least 9 types of bacteria from the three classification groups including proteobacteria, cyanobacteria, and actinobacteria.

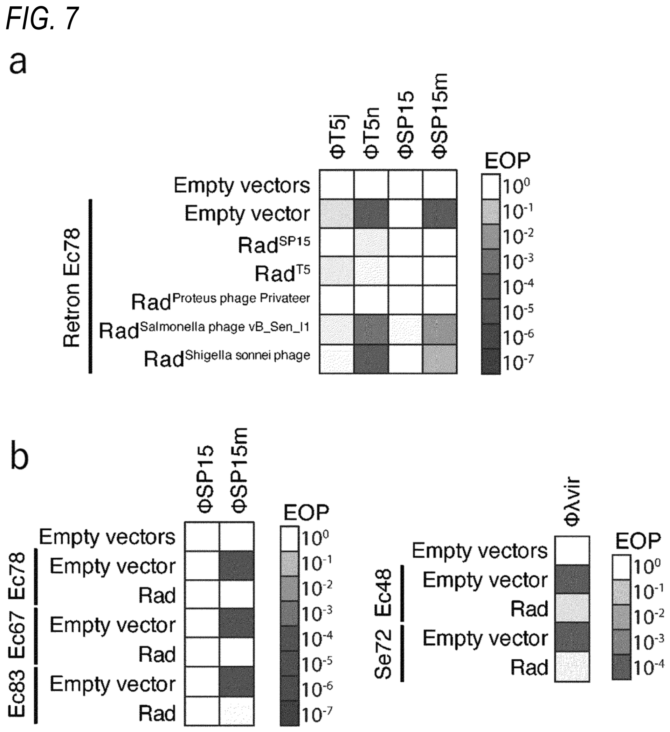

Recombinant plasmids for expressing Rad homologues from other phages infecting different bacterial species were prepared in the same manner as in Test Example 1. The recombinant plasmid was introduced into Escherichia coli having a Retron, phages were caused to infect, and whether the infection was established was examined by spot assay in the same manner as in Test Example 1. The results are shown in a of FIG. 7.

As shown in a of FIG. 7, it was found that the Rad homologue of the Proteus mirabilis phage (RadProteus phage Privateer, SEQ ID NO: 13) can protect the phage from Retron similarly to the SP15 phage-derived Rad. In addition, the Rad homologue (SEQ ID NO: 14) derived from the Shigella sonnei phage (RadShigella sonnei phage) and the Rad homologue (SEQ ID NO: 12) derived from the Salmonella phage vB_Sen_I1 (RadSalmonella phage vB_Sen_I1) showed moderate protection.

### (3) Inhibition of Retron Including Ec48 and Se72 by Rad

A recombinant plasmid expressing SP15 phage-derived Rad was prepared in the same manner as in Test Example 1, the recombinant plasmid was introduced into Escherichia coli having a Retron, the phage was caused to infect, and whether infection was established was examined. The results are shown in b of FIG. 7.

As shown in b of FIG. 7, Rad exhibited a wide range of inhibitory effects on Retron including Ec48 and Se72.

### (4) Analysis of Rad for Anti-Retron Activity

As a result of searching for protein domains using Pfam-A_35, COG_KOG_v 1.0, and PHROGs_4 databases, Rad had a Toprim domain (phrog_12622). The amino acid residues at positions 98 to 198 from the N-terminus were similar to Toprim of rRNA maturation endonuclease (COG1658).

In order to evaluate which region of the Rad protein is important for anti-Retron activity, various Rad mutants were prepared by introducing amino acid mutations into various sites (R13E, P33T, I88T, D135H, and E156H) which are stored in another Rad and very similar to proteins obtained from a database.

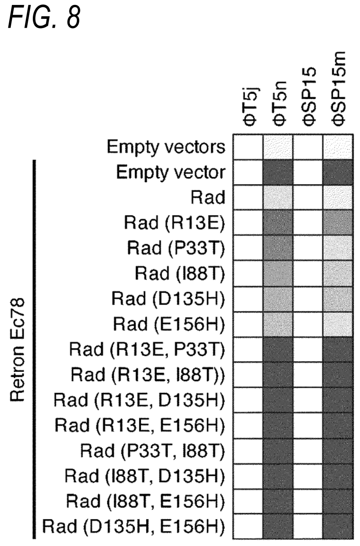

Recombinant plasmids for expressing the obtained various Rad mutants were prepared in the same manner as in Test Example 1. The recombinant plasmid was introduced into Escherichia coli having a Retron, phages were caused to infect, and whether the infection was established was examined by spot assay in the same manner as in Test Example 1. The results are shown in FIG. 8.

As shown in FIG. 8, it was found that the activity of Rad to inhibit Retron was slightly reduced by mutation of the selected amino acid residue. It was also found that when two mutants of the selected amino acids were introduced together, the Rad activity was completely lost regardless of the location.

### [Test Example 3] Analysis of Effector Protein PtuAB in Retron

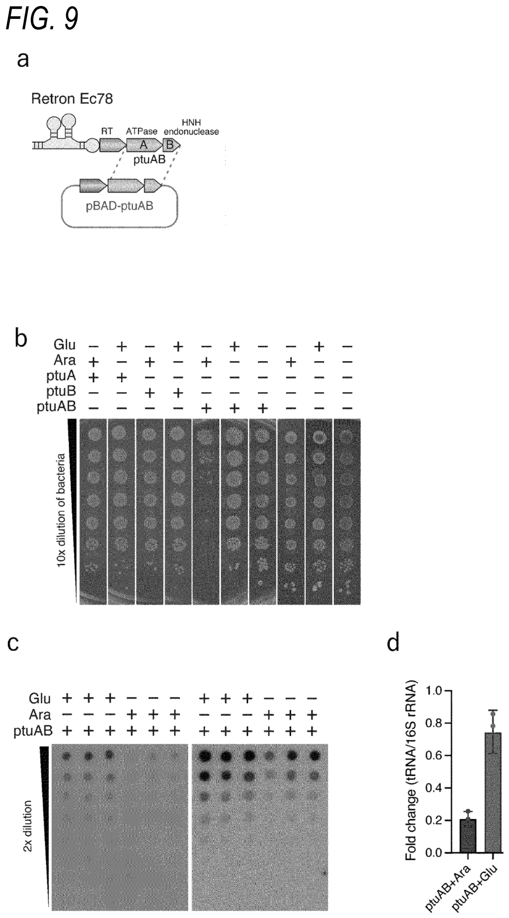

tRNA-Tyr is an intracellular target of the Retron Ec78 effector protein. The phage can evade the Retron Ec78 by phage-derived tRNATyr (tRNATyr SP15) of F6 DRR, and therefore, tRNATyr of the bacterium was presumed to be a target of the Ec78 effector protein. Effector proteins in Retron Ec78 are PtuA, which is a protein having an ATPase domain, and PtuB, which is an HNH endonuclease. a of FIG. 9 is a simplified diagram of a method for evaluating an intracellular target of ptuAB of Retron Ec78. The Ec78 effector protein (ptuAB) was cloned into a plasmid under the pBAD promoter together with PtuA or PtuB alone or PtuAB. Glucose was used to block the pBAD promoter, and arabinose was used to induce the promoter.

A recombinant plasmid was prepared by inserting a nucleotide sequence encoding PtuA, PtuB, or PtuAB (PtuA: SEQ ID NO: 20, PtuB: SEQ ID NO: 21) into an expression vector pBAD-ptuAB. The recombinant plasmid is a vector into which expression of PtuA, PtuB, or PtuAB is introduced by adding arabinose to a medium, and the expression is not introduced by adding glucose to the medium.

The recombinant plasmid was introduced into E. Coli having Retron Ec78, and the decrease in the expression level of tRNA-Tyr was evaluated by the cytotoxicity test, dot blot RNA hybridization, and tRNA-sequencing.

The cytotoxicity test was performed according to the following procedure. PtuAB was cloned into a plasmid pKLC23 under the pBAD-inducible promoter using a Hifi NEBuilder assembly. Escherichia coli DH10B was transformed with this plasmid construct using a heat shock protocol. A single bacterial colony having PtuAB is inoculated into 2 ml of LB containing chloramphenicol, and the mixture was cultured overnight at 37°C while shaking at 200 rpm (until OD595 reaches 5). The overnight culture was continuously diluted 8 times (10 times) stepwise with LB. The culture diluent was spotted in an amount of 3 µl on an LB plate not containing appropriate antibiotics and arabinose, glucose, or saccharides. Then, the culture solution was incubated at 37°C overnight.

The dot blot RNA hybridization was performed according to the following procedure. An overnight culture solution of Escherichia coli DH10B harboring the PtuAB plasmid was inoculated into 10 ml of LB supplemented with chloramphenicol (20 µg/ml) until OD600 reached 0.3. Thereafter, induction was performed with 0.2% arabinose (to induce PtuAB expression) or 0.2% glucose (to inhibit PtuAB expression), followed by incubation for 2 hours at 37°C with shaking at 200 rpm. After the incubation, the culture solution was centrifuged at 6000g for 5 minutes, and the pellets were washed twice with a PBS buffer. Total RNA was extracted using a miRNA separation kit (QIAGEN, catalog number: 217004). This total RNA was used for dot blot RNA hybridization.

The tRNA-sequencing method was performed according to the following procedure. Total RNA of each sample was quantified by NanoDrop ND-1000. The tRNA was purified from the total RNA sample, and m1A and m3C were demethylated and partially hydrolyzed according to the Hydro-tRNAseq method. Thereafter, the partially hydrolyzed and rephosphorylated tRNA fragment was converted into small RNA sequencing libraries using NEBNext (registered trademark) Multiplex Small RNA Library Prep Set for Illumina (registered trademark) kit (New England Biolabs). The size of the PCR amplified fragment of 140 bp to 155 bp (corresponding to the size range of the tRNA fragment of about 19 nt to 35 nt) was selected. The generated tRNA-seq library was checked for eligibility using Agilent 2100 BioAnalyzer, and absolute quantification was performed. Then, the libraries were uniformly mixed, and 50 cycles of sequencing were performed in the Illumina NextSeq 500 system according to instructions of the manufacturer using NextSeq 500/550 High-Output v2 kit (75 cycles).

The quality of the sequence was checked by FastQC software, and trimmed reads (passing an Illumina quality filter and trimming 3'-adaptor bases using cutadapt) were aligned with cytoplasmic mature tRNA sequences obtained from the GtRNAdb BWA6 software. In the alignment of tRNAs, the maximum mismatch was 27. The tRNA expression profiles were calculated based on the uniquely mapped reads and the including mapped reads, respectively. Using the R-package edge R8, tRNAs having different expression levels were screened based on the count value. In the R or Python environment, principal component analysis (PCA), correlation analysis, hierarchical clustering, a scatter diagram, Venn plots, and Volcano plots were performed, and statistical calculation and graphics were performed.

b of FIG. 9 shows results of the cytotoxicity test, and c and d of FIG. 9 show results of the dot blot RNA hybridization. As shown in b of FIG. 9, the induction of PtuAB promotes the stop of the growth of bacteria. The induction of PtuA or PtuB alone was not toxic to bacteria, but induction of both (PtuAB) was toxic. In addition, as shown in c and d of FIG. 9, tRNATyr was significantly low in the bacteria in which PtuAB was expressed.

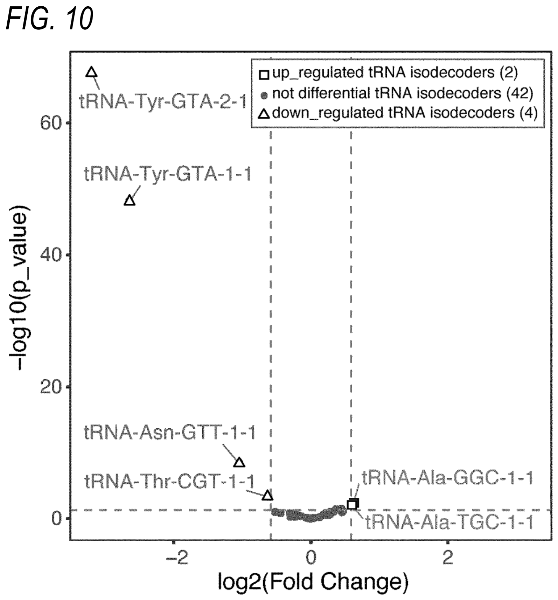

FIG. 10 shows results of the tRNA-sequencing. As shown in FIG. 10, it was found that tRNATyr is significantly controlled when PtuAB is induced.

### [Test Example 4] Inhibition of Retron by Replenishment of tRNA

The tRNA-Tyr was cloned from various phages and inserted downstream of a ΦtRNA-Tyr promoter (SP15 phage-derived tRNA-Tyr promoter, SEQ ID NO: 78) in the vector pKLC23 to prepare a recombinant plasmid. The recombinant plasmid was introduced into a bacterium having Retron Ec78 to perform transformation, and then the bacterium was infected with a phage to examine, by a spot assay, whether the infection was established.

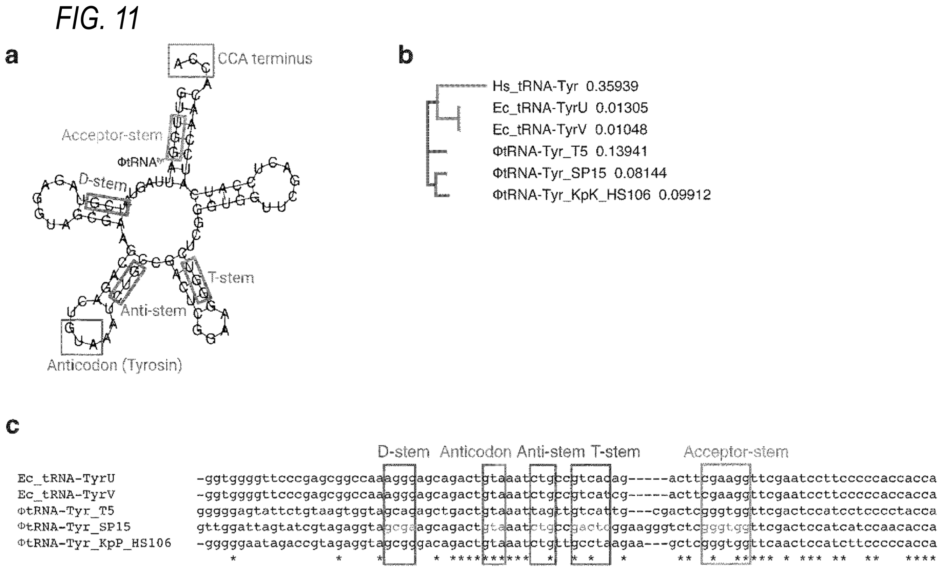

a of FIG. 11 shows a structure of SP15 phage-derived tRNA-Tyr predicted by RNAFold3. In order to evaluate the effect of the mutation on the restoration of phage from retron Ec78, a sequence mutation and a structural mutation were introduced into a tRNA. These mutations are CCA-terminus (CCA into AAA), acceptor-stem (UGG into AAA), D-stem (UGG into AAA), anti-stem (GUC into AAA), and T-stem (GGU into AAA).

b of FIG. 11 shows a tRNA-Tyr phylogenetic tree, and c of FIG. 11 shows sequence alignments of various kinds of tRNA-Tyr. The sequences of tRNA-Tyr are shown in Table 3.

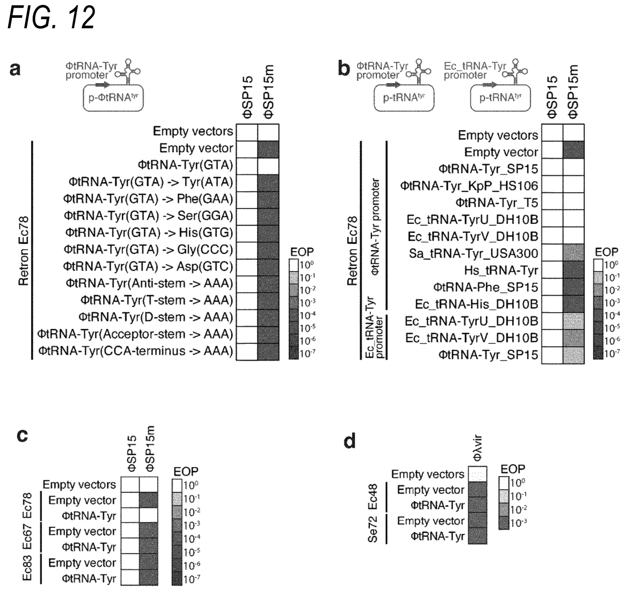

The results of the spot assay are shown in a to d of FIG. 12. As shown in a of FIG. 12, it was found that the mutation of tRNATyr lost the ability of tRNA-Tyr that neutralizes the retron defense regardless of the location where the mutation occurred.

As shown in b of FIG. 12, tRNATyr derived from different phages (T5 and Podophage-2) or Escherichia coli relieved the phage from the retron Ec78. In particular, Ec_tRNA_Tyr was able to rescue the phage in the same manner as tRNA_Tyr_SP15 when the SP15 tRNA promoter was used, but the Retron anti-phage defense activity was still observed when the E. coli tRNA promoter (SEQ ID NO: 79) was used. Other tRNAs (tRNAPhe or tRNAHis) and tRNATyr derived from human (Hs_tRNA_Tyr) or tRNATyr derived from Staphylococcus aureus (Sa tRNA_Tyr_USA300) did not relieve phages from retron Ec78.

As shown in c and d of FIG. 12, tRNA-Tyr specifically rescued the phage from Retron Ec78. The co-expression of tRNA-Tyr and another retron was not able to relieve the phage SP15m from Retron Ec67, Retron Ec78, and Retron Ec83, and similarly, tRNA-Tyr was not able to relieve the phage λvir from Retron Ec48 and Retron Se72.

### [Test Example 5] Cloning of Retron-Sensitive Component

A phage mix in which phages having various mutations were mixed was caused to infect bacteria having a Retron, phage genome sequencing was performed on the phage that has infected the bacteria, and genes with mutations were examined.

### (1) Isolation of Retron-escape Phage

Three types of Retron, Ec67 (pLG006), Ec78 (pLG008), and Ec83 (pNNK3), were used for screening of escaper phages. For Retron Ec67, the phage T5n, the phage SP15m, or the phage T2 was co-cultured separately with bacteria harboring pLG006 at MOI = 1 in 4 ml of LB supplemented with an antibiotic and 1 mM CaCl₂, followed by incubation overnight at 37°C while shaking at 200 rpm. For Retron Ec78 or Retron Ec83, T5n and SP15m were used. After overnight incubation, the lysate was centrifuged at 5000g for 10 minutes, and the supernatant was filtered through a 0.2 µM filter to remove remaining bacteria. The obtained solution was used as a phage mixing solution because the solution may contain wild-type phages and retron-escape phages.

In order to examine the ability of the phage to escape from the Retron anti-phage defense system, a double-layer plaque assay was performed with bacteria having Retron or a negative control. The same amounts (100 µl each) of the bacteria cultured overnight and the phage mixture were mixed with 4 mL of LB top agar supplemented with an antibiotic, and the mixture was mixed by vortex and then added to an LB plate. The plate was incubated at 37°C overnight. The efficiency of plating (EOP) was measured and compared between a defense strain and a NC strain. A phage that forms a single plaque with a bacterium harboring Retron was regarded as a Retron-escape mutant phage (hereinafter, also abbreviated as a mutant phage).

In order to purify the mutant phage, a single plaque formed in the Retron-harboring bacteria was isolated and added to a liquid culture of the Retron-harboring bacteria cultured in 1 mL of LB supplemented with CaCl₂ so as to reach OD600 of 0.3, and the mutant phage was further proliferated. After the phages were incubated with the bacteria at 37°C and 200 rpm for about 3 hours, 9 mL of a bacterial culture solution proliferated to OD600 of 0.3 with LB supplemented with CaCl₂ was added, followed by further incubating at 37°C for about 3 hours while shaking at 200 rpm, the thus-obtained solution was centrifuged at 5000g for 10 minutes, and the supernatant was filtered with a 0.2 µM filter to remove remaining bacteria.

Thereafter, the phage titer was checked by a plaque assay using a negative control strain, and when the phage titer was less than 10⁷ pfu/ml, the phage titer was increased by proliferation in liquid culture or by using plate lysate 2 in Retron-harboring cells. For the plate lysate, the phage lysate containing 10³ to 10⁵ plaque-forming units (pfu) was mixed with 100 µl of Retron-carrying cells, followed by leaving at room temperature for 10 minutes, then 4 mL of an LB top agar supplemented with an antibiotic was added, and the mixture was poured into an LB plate. The phages were incubated with bacteria overnight at 37°C so as to lyse the cells. Thereafter, the entire upper layer was scraped into 5 mL of an SM buffer, followed by holding at room temperature for 1 hour. During this period, the phages were mixed several times with a vortex and released from the agar to the SM buffer. The phages were centrifuged at 5000g for 10 minutes to remove agar and bacterial cells, and the supernatant was filtered through a 0.2 µM filter.

### (2) Nucleotide Sequence Determination and Genome Analysis of Escaper Phage Mutant

For DNA extraction, a high-titer phage lysate (> 10⁷ pfu/ml) from ancestral phage mutants and the isolated phage mutant was used. The phage lysate (500 µl) was treated with DNase-I (Takara Bio Inc.) and RNAse A (Takara Bio Inc.) added to have final concentrations of 3 U/ml and 20 ng/ml, respectively, followed by incubating at 37°C for 1 hour, and bacterial DNA and RNA were removed. A phage DNA extraction kit (Norgen Biotex Corp.) was used according to a protocol of the manufacturer. The whole genome was determined using the whole genome analysis service of Macrogen Japan Corp. Bioinformatics analysis was performed using CLC Genomic workbench (QIAGEN, USA). Silent mutations in a protein-coding region were also ignored.

### (3) Evaluation of Retron Activation by Phage Gene

Genes that had been fully mutated in escaper phages were selected, and were further evaluated for the role in Retron activation. A plasmid containing a phage gene selected under the inducible promoter ATc of pKLC831 was introduced into a Retron-harboring bacterium or a negative control cell having an empty vector. After culturing overnight, at least four colonies were inoculated into 2 mL LB supplemented with ampicillin (100 µg/mL) + chloramphenicol (20 µg/mL) + 0.2% glucose, and cultured overnight at 37°C while shaking at 200 rpm. The overnight cultures were continuously diluted 8 times (10 times) stepwise with LB. The culture diluent was spotted in an amount of 3 µl on an LB plate not containing appropriate antibiotics and arabinose, glucose, or saccharides. Then, the culture solution was cultured at 37°C overnight. The DenB gene shows slight toxicity when overexpressed at the maximum inducer level, and therefore, the inducer level was lowered to the extent that the expression did not show toxicity in control cells.

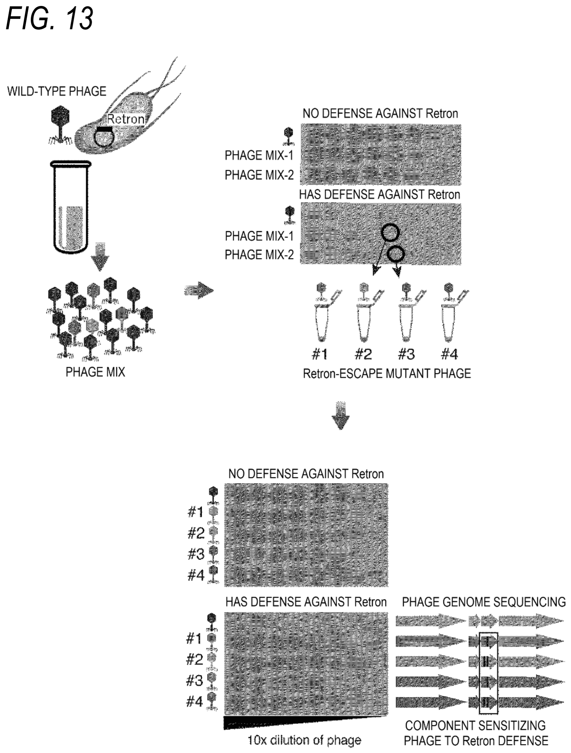

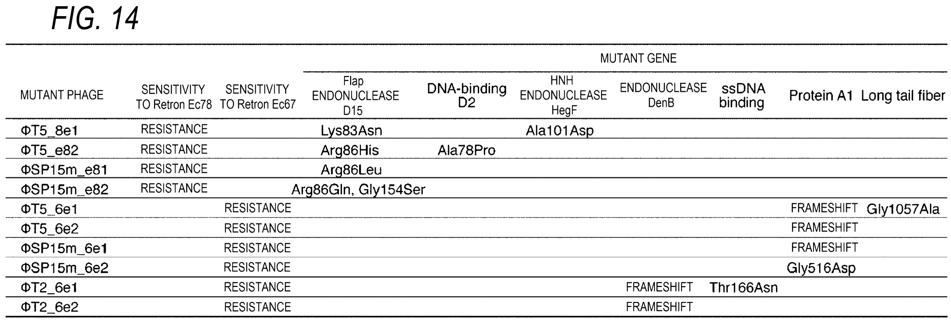

FIG. 13 is a schematic diagram showing an outline of phage genome sequencing, and FIG. 14 shows the results. The sequence of the genome of each phage shown in FIG. 14 is shown in Table 6.

### [Table 6]

**Table 6**

| SEQ ID NO: | Gene name | Derivatives |
|---|---|---|
| 80 | Genome of T5 8e1 | T5 8e1 phage |
| 81 | Genome of T5 8e2 | T5 8e2 phage |
| 82 | Genome of SP15m 8e1 | SP15m 8e1 phage |
| 83 | Genome of SP15m 8e2 | SP15m 8e2 phage |
| 84 | Genome of T2 6e1 | T2 6e1 phage |
| 85 | Genome of T2 6e2 | T2 6e2 phage |
| 86 | Genome of SP15m 6e1 | SP15m 6e1 phage |
| 87 | Genome of SP15m 6e2 | SP15m 6e2 phage |
| 88 | Genome of T2 6e1 | T2 6e1 phage |
| 89 | Genome of T2 6e2 | T2 6e2 phage |

Next, a genome sequence of each mutant phage was compared with a sequence of a parent phage.

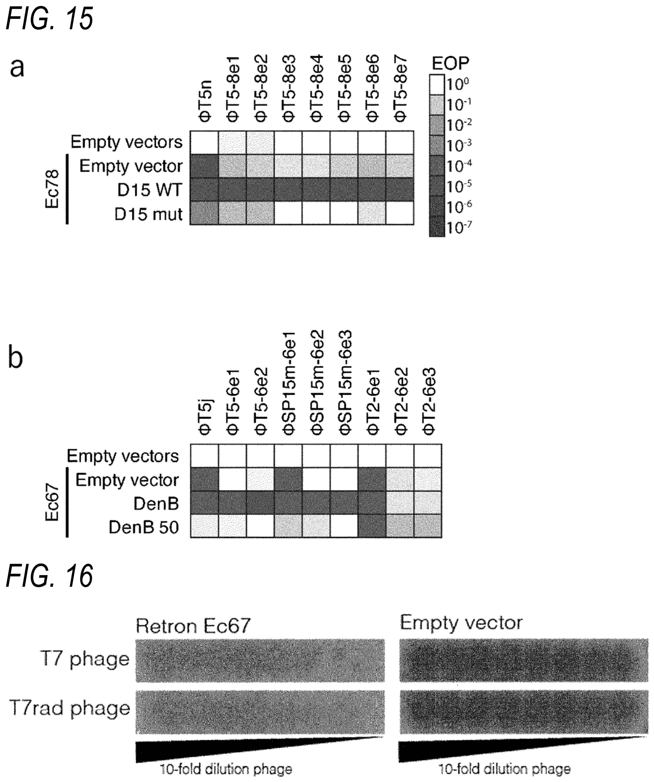

Regarding Retron Ec78, it was found that there was a mutation in the gene encoding exonuclease D15 in all mutants of T5n (7 mutants) and ΦSP15m (4 mutants). The D15 protein is a T5-derived protein that catalyzes 5'-exonucleolysis degradation and structure-specific endonucleolysis degradation of a DNA branched nucleic acid molecule (Pickering, T. J. et al., Journal of Biological Chemistry 274, 17711-17717, 1999; Garforth, S. J. et al., Proceedings of the National Academy of Sciences 96, 38-43, 1999; Feng, M. et al., Nat Struct Mol Biol 11, 450-456, 2004). The results of spot assay performed by co-expressing D15 protein (nucleotide sequence: SEQ ID NO: 90, amino acid sequence: SEQ ID NO: 91, UniProt ID: AAU05267.1) and Retron Ec78 are shown in a of FIG. 15.

As shown in a of FIG. 15, the defense activity of Ec78 against mutant T5n phage and the like was recovered, but there was no toxicity to bacteria. These results suggest that the defense activity of Rectron Ec78 may be caused not only by the D15 protein but also by other unknown factors.

Regarding Retron Ec67, there were mutations in DenB and protein A1 in all of T2, T5n and SP15m phages, and therefore, these genes were presumed to be genetic factors for Ec67 activation. The protein A1 and DenB are both involved in DNA degradation. The protein A1 is involved in the degradation of the host DNA and the isolation of the host gene (Stokar-Avihail, A. et al., BiorXIv, 2022, doi: 10.1101/2022, 08.27, 505566; McCorquodale, D. J. et al., J Virol. 40, 958-962, 1981; Beckman, L. D. et al., J Mol Biol, 62, 551-564, 1971). On the other hand, the DenB protein is considered to be harmful to dC-specific cleavage of single-stranded DNA and replication of a dC-containing DNA of the host (Ohshima, H. et al., Nucleic AcidsRes 35, 6681-6689, 2007; Hirano, N. et al., Nucleic Acids Res, 34, 4743-4751, 2006).

In order to prove that the DenB and protein A1 are involved in the activation of Retron Ec67, each gene was cloned into a bacterium having Ec67 under the inducible promoter. The protein A1 had high toxicity, and therefore, the effect of co-expression of the protein A1 and Retron Ec67 was not able to be observed. DenB (nucleotide sequence: SEQ ID NO: 92, amino acid sequence: SEQ ID NO: 93, UniProt ID: AYD82860.1) and Retron Ec67 were co-expressed, and the results of the spot assay are shown in b of FIG. 15.

As shown in b of FIG. 15, the co-expression of DenB and Retron Ec67 significantly inhibited the proliferation of bacteria. Although the T5n and SP15m phages had no DenB homologue protein, the complementarity of DenB recovered the defense activity of Ec67 not only for the T2 mutant but also for the T5n and SP15m phages. Therefore, it was suggested that the defense of Retron Ec67 was not caused by the denB protein itself but by the denB activity.

### [Test Example 6] Preparation of T7 Phage with Retron Inhibitor Rad

In order to introduce Rad into the T7 phage genome, the following synthesis approach was performed. A fragment of the phage genome having a homologous arm of about 40 bp was synthesized by PCR. The primers used for PCR are shown in Table 7.

### [Table 7]

**Table 7**

| SEQ ID NO: | Names | PCR product | Template DNA |
|---|---|---|---|
| 64 | forward primer Rad-T7 | SP15 | To insert Rad into T7 |
| 65 | reverse primer Rad-T7 | SP15 | |
| 66 | forward primer T7F1 | T7 | T7 fragment1 |
| 67 | reverse primer T7F1-Rad | T7 | |
| 68 | forward primer T7F2-Rad | T7 | T7 fragment2 |
| 69 | reverse primer T2F2 | T7 | |
| 70 | forward primer T7F3 | T7 | T7 fragment3 |
| 71 | reverse primer T7F3 | T7 | |
| 72 | forward primer T7F4 | T7 | T7 fragment4 |
| 73 | reverse primer T7F4 | T7 | |
| 74 | forward primer T7F5 | T7 | T7 fragment5 |
| 75 | reverse primer T7F5 | T7 | |
| 76 | forward primer T7F6 | T7 | T7 fragment6 |
| 77 | reverse primer T7F6 | T7 | |

The synthetic fragment of Rad or tRNA was prepared by PCR or DNA gBlock containing a genomic fragment of a target phage into which Rad or tRNA was inserted and a 40 bp homologous arm. A phage and a synthetic DNA of Rad or tRNA were mixed with a NEBuilder master mix using a NEBuilder Hifi DNA assembly (manufactured by New England Biolab) to prepare a circular synthetic phage genome according to a protocol of a manufacturer.

The circular genome was electroporated into Escherichia coli. Thereafter, the cells for which the electroporation were performed were incubated at 37°C for 30 minutes while shaking at 200 rpm. After culturing, 100 µl of cells were mixed with LB agar (LB, 0.5% agarose, with 1 mM CaCl₂ added) and the mixture was vortexed and finally poured into LB agar. After culturing at 25°C overnight, the Rad-carrying mutant T7 phage (T7 rad) was purified by picking up a single plaque with a toothpick and further proliferated using Escherichia coli DH10B. The activity of Rad at T7 rad was evaluated by the spot assay of DH10B regardless of the presence or absence of retron Ec67. The results are shown in FIG. 16.

As shown in FIG. 16, it was found that by incorporating the gene encoding Rad on the T7 phage genome, the infectivity to bacteria having a Retron anti-phage defense system can be obtained.

### [Test Example 7] Homologous Recombination of Retron Inhibitor Rad or tRNA to SP15m Phage

Rad or tRNA-Tyr-SP15 was introduced into a SP15m phage using homologous recombination. Synthetic Rad DNA or tRNA was prepared using PCR. The primers used for PCR are shown in Table 8.

### [Table 8]

**Table 8**

| SEQ ID NO: | Names | Object of amplification | Template DNA |
|---|---|---|---|
| 94 | forward primer_tRNATyr-DRRmSP | Insertion of tRNA-Tyr-SP15 into SP15m | SP15 |
| 95 | reverse primer tRNATyr-DRRmSP | Insertion of tRNA-Tyr-SP15 into SP15m | SP15 |
| 96 | forward primer Rad-DRRmSP | Insertion of Rad into SP15m | SP15 |
| 97 | reverse primer Rad-DRRmSP | Insertion of Rad into SP15m | SP15 |
| 98 | forward primer DRRSP15check | Checking of insertion in SP5m | SP15m |
| 99 | reverse primer DRRSP15check | Checking of insertion in SP5m | SP15m |

Genomic DNA of SP15 was extracted using a phenol-chloroform method. A phage lysate (0.5 ml) containing 10¹⁰ or more plaque-forming units per milliliter was treated with 12 units of DNAse I (TAKARA) and 50 µg of RNAse A (TAKARA) at 37°C for 1 hour. After the treatment, an equal amount of a TE-saturated phenol: chloroform (1:1) solution was added to the phage lysate, the mixture was vortexed for 30 seconds and centrifuged at 12000g for 3 minutes, and phenol: chloroform extraction was performed.

The supernatant was collected, and the phenol: chloroform extraction was repeated at least three times. To the supernatant was added 1.5-fold amount of ethanol to precipitate a DNA, followed by incubation at -20°C for 30 minutes or longer. The DNA was collected by centrifugation at 14000 rpm for 30 minutes at 4°C and washing twice with 70% ethanol. Finally, the DNA was dried at room temperature and eluted with a TE buffer (10 mM Tris, pH 8, 1 mM EDTA, pH 8.0).

Homologous recombination was achieved using Bacteriophage Recombineering of Electroporated DNA (BRED) protocol. The PCR product and the genome of SP15m were simultaneously electroporated into Escherichia coli, followed by incubation at 37°C while shaking at 200 rpm for 1 hour.

Thereafter, centrifugation is performed at 8000 rpm, 100 µl of the supernatant is mixed with bacteria carrying Retron with LB top agar, and the mixture was finally poured into an LB plate. After culturing at 37°C overnight, a mutant phage carrying Rad (SP15m-Rad) or a mutant phage carrying tRNA-Tyr (SP15m-tRNATyr) was purified by picking up a single plaque with a toothpick, and further proliferated using Escherichia coli expressing Retron Ec78. The activity of Rad or tRNATyr was evaluated by a spot assay.

As a result of the spot assay, it was found that the infectivity to bacteria having a Retron anti-phage defense system can be obtained by incorporating Rad or tRNATyr into the SP15m phage.

### [Test Example 8] Synthesis of T7 Phage with Retron Inhibitor (Rad)

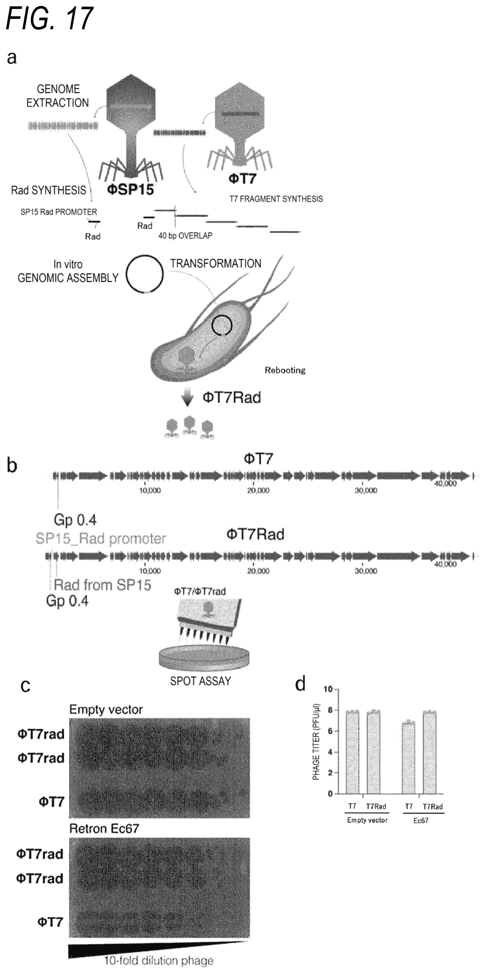

As shown in a of FIG. 17, the T7 phage with a Retron inhibitor (Rad) was synthesized according to the following procedure. A phage was synthesized by adding a Rad fragment to the genome of the T7 phage. A DNA region encoding Rad was amplified downstream of the tRNATyr promoter of the T7 genome using KOD FX Neo (TOYOBO, catalog number: #KFX-201), and the DNA region was circularly reconstructed using NEBuilder (registered trademark) Hifi DNA assembly (New England Biolabs, catalog number #E2621).

As shown in b of FIG. 17, Rad was designed to be inserted upstream of gp 0.4 of the T7 phage. The T7 phage DNA encoding T7-Rad was electroporated into Escherichia coli HST08 (Takara Bio, Tokyo, Japan), and the Escherichia coli HST08 was incubated at 37°C for 20 minutes and mixed with DH10B cultured overnight in 4 mL of LB top agar (LB, 0.5% agarose, 1 mM CaCl₂), followed by pouring into an LB plate to form plaques (b of FIG. 17).

A single plaque of T7-Rad (T7 phage-harboring Rad) was obtained and added to 1 mL of DH10B (OD600 = 0.3) proliferated in LB supplemented with CaCl₂. Shaking was performed at 37°C and 200 rpm for about 3 hours, and 9 mL of DH10B (OD600 = 0.3) was further added. The obtained mixture was further incubated at 37°C while shaking at 200 rpm for about 3 hours. Thereafter, the lysate was centrifuged at 5000 ×g for 10 minutes, and the supernatant was filtered through a 0.2 µM filter to remove remaining bacteria. c of FIG. 17 shows results of the spot assay, and d of FIG. 17 shows measurement results of the phage titer.

As shown in c and d of FIG. 17, it was found that the T7 phage with a Retron inhibitor (Rad) was not inhibited by Retron.

### [Test Example 9] Inhibition of Defense System Other than Retron by Rad

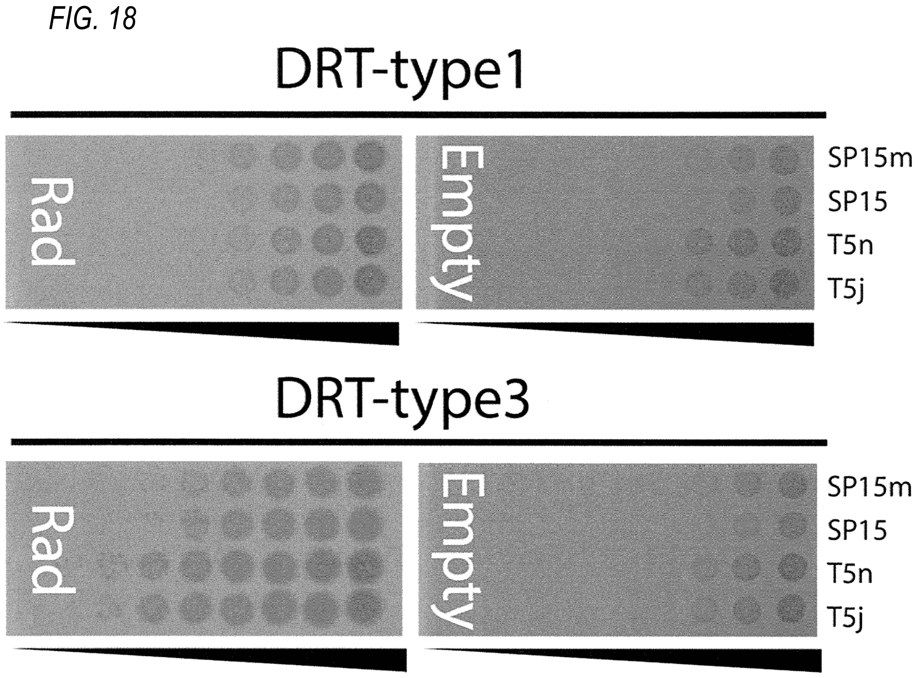

Rad was expressed in Escherichia coli DH10B strains harboring defense systems (DRT-type 1 and DRT-type 3), and the strains were cultured in a layer agar medium. SP15m, SP15, T5n, and T5j phages were spotted and cultured on the plate. FIG. 18 shows the results of the spot assay.

As shown in FIG. 18, it was found that Rad inhibits DRT-type1 and DRT-type3, which are defense systems other than Retron.

### [Test Example 10] Synthesis of T7 Phage with tRNALys

A phage was synthesized by adding the tRNALys fragment to the T7 phage genome. A DNA region encoding tRNALys was amplified downstream of the tRNATyr promoter of the T7 genome using KOD FX Neo (TOYOBO, catalog number: #KFX-201), and the DNA region was circularly reconstructed using NEBuilder (registered trademark) Hifi DNA assembly (New England Biolabs, catalog number #E2621). The tRNALys was designed to be inserted upstream of gp 0.4 of the T7 phage. The tRNAs used are shown in Table 9.

### [Table 9]

**Table 9**

| SEQ ID NO: | tRNA | Derivatives |
|---|---|---|
| 100 | tRNA-LysDH10B | *Escherichia coli* DH10B |
| 101 | tRNA-AsnDh10B | *Escherichia coli* DH10B |
| 102 | tRNA-LvsSP15 | phage SP15 |
| 103 | tRNA-AsnSP15 | phage SP16 |

The T7 phage DNA encoding T7-tRNALys was electroporated into Escherichia coli HST08 (Takara Bio, Tokyo, Japan), and the Escherichia coli HST08 was incubated at 37°C for 20 minutes and mixed with DH10B cultured overnight in 4 mL of LB top agar (LB, 0.5% agarose, 1 mM CaCl₂), followed by pouring into an LB plate to form plaques.

A single plaque of T7-tRNALys (T7 phage-harboring tRNALys) was obtained and added to 1 mL of DH10B (OD600 = 0.3) proliferated in LB supplemented with CaCl₂. Shaking was performed at 37°C and 200 rpm for about 3 hours, and 9 mL of DH10B (OD600 = 0.3) was further added. The obtained mixture was further incubated at 37°C while shaking at 200 rpm for about 3 hours. Thereafter, the lysate was centrifuged at 5000 ×g for 10 minutes, and the supernatant was filtered through a 0.2 µM filter to remove remaining bacteria.

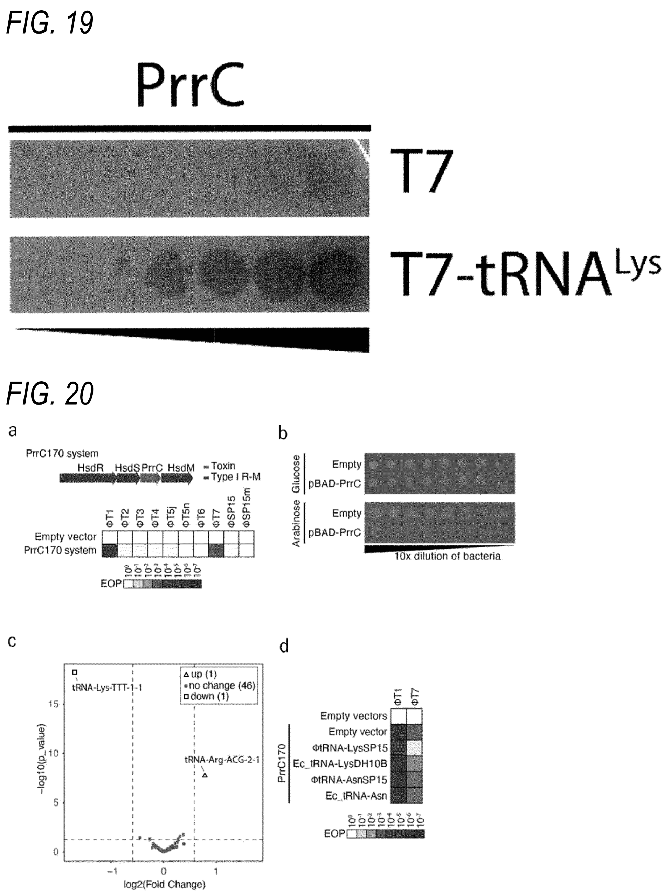

In order to evaluate the effect of tRNALys expression in defense system evasion, a spot assay was performed on a bacterium harboring PrrC 170 anticodon nuclease using T7 wild-type and T7-tRNALys. The results of the spot assay are shown in FIG. 19.

As shown in FIG. 19, it was found that T7 phage with tRNALys was not inhibited by PrrC.

### [Test Example 11] Inhibition of PrrC Anti-phage Defense System by tRNA

In order to construct a plasmid having a PrrC defense system, a PrrC TA system including four genes (HsdR, HsdS, PrrC, and HsdM) containing 300 nucleotides before a start codon of HsdR was cloned into a plasmid pLG001. The primers used in PCR are shown in Table 10, and PrrC, HsdR, HsdS, and HsdM are shown in Table 11.

### [Table 10]

**Table 10**

| SEQ ID NO: | Primer name | Template | PCR product |
|---|---|---|---|
| 104 | fPrrC170 | Genome of bacteria JBEAAAI-19-0008 | PrrC system |
| 105 | rPrrC170m | Genome of bacteria JBEAAAI-19-0008 | PrrC system |
| 106 | fPrrC170m | Genome of bacteria JBEAAAI-19-0008 | PrrC system |
| 107 | rPrrC170 | Genome of bacteria JBEAAAI-19-0008 | PrrC system |
| 108 | FpPrrC170 | pLG001 | plasmid backbone |
| 109 | RpPrrC170 | pLG001 | plasmid backbone |
| 110 | fPrrC170-pK | Genome of bacteria JBEAAAI-19-0008 | PrrC toxin |
| 111 | fPrrC170-pK | Genome of bacteria JBEAAAI-19-0008 | PrrC toxin |
| 112 | FpK-PrrC170 | pKLC23 | plasmid backbone |
| 113 | RpK-PrrC170 | pKLC23 | plasmid backbone |

### [Table 11]

**Table 11**

| SEQ ID NO: | Names | Derivatives |
|---|---|---|
| 114 | PrrC170 system sequence | JBEAAAI-19-0008 |
| 115 | PrrC170 toxin | JBEAAAI-19-0008 |
| 116 | HsdR | JBEAAAI-19-0008 |
| 117 | HsdS | JBEAAAI-19-0008 |
| 118 | HsdM | JBEAAAI-19-0008 |

a of FIG. 20 shows a heat map showing an anti-phage function of the PrrC170 anticodon nuclease. The assembly of the plasmid was performed using NEBuilder (registered trademark) Hifi DNA assembly (New England Biolabs, USA, catalog number #E2621). The Escherichia coli DH10B was transformed with the plasmid construct using a heat shock method.

b of FIG. 20 shows results of the spot assay (toxicity assay), c of FIG. 20 shows results of the tRNA-sequencing, and d of FIG. 20 shows results of the spot assay (phage infection experiment).

As shown in b of FIG. 20, proliferation termination was observed in the bacterium expressing a PrrC toxin. c of FIG. 20 shows expression of a tRNA in a bacterium harboring PrrC. d of FIG. 20 shows that a PrrC170 anti-phage defense system malfunctions in a bacterium expressing tRNA-Lys. From these results, it was found that the tRNA can also inhibit anti-defense systems other than Retron.

Although various embodiments have been described above with reference to the drawings, it is needless to say that the present invention is not limited to such examples. It is clear that those skilled in the art can conceive various modifications or alterations within the scope of the claims, and it is understood that these modifications or alterations also naturally fall within the technical scope of the present invention. In addition, the components in the above-described embodiments may be freely combined without departing from the gist of the invention.

The present application is based on a Japanese Patent Application (Patent Application No. 2023-041423) filed on March. 15, 2023, the contents of which are incorporated herein by reference.

### INDUSTRIAL APPLICABILITY

The recombinant phage of the present invention is highly likely to be used as an active ingredient of an antibacterial agent. The recombinant phage can also be used for editing bacterial flora. The recombinant phage can also be used to remove bacteria from food, as a disinfectant, and as an agricultural chemical. The recombinant phage can be used for biotechnology using Retron. The recombinant phage can also be used to stop a reaction of gene recording. PtuA/B can be used as a specific RNA cleavage enzyme for tRNA-tyr.

### SEQUENCE LISTING FREE TEXT

SEQ ID NO: 1: Nucleotide sequence of T5n phage genome
SEQ ID NO: 2: Nucleotide sequence of T5j phage genome
SEQ ID NO: 3: Nucleotide sequence of SP15m phage genome
SEQ ID NO: 4: Nucleotide sequence of SP15 phage genome
SEQ ID NO: 5: Nucleotide sequence of Rad-SP15
SEQ ID NO: 6: Nucleotide sequence of Rad-T5j
SEQ ID NO: 7: Nucleotide sequence of Rad-Salmonella vB Sen_I1
SEQ ID NO: 8: Nucleotide sequence of Rad-Proteus phage Privateer
SEQ ID NO: 9: Nucleotide sequence of Rad-Shigella Sonnei phage
SEQ ID NO: 10: Amino acid sequence of Rad-SP15
SEQ ID NO: 11: Amino acid sequence of Rad-T5j
SEQ ID NO: 12: Amino acid sequence of Rad-Salmonella vB Sen_I1
SEQ ID NO: 13: Amino acid sequence of Rad-Proteus phage Privateer
SEQ ID NO: 14: Amino acid sequence of Rad-Shigella Sonnei phage
SEQ ID NO: 15: Nucleotide sequence of tRNA-Tyr T5
SEQ ID NO: 16: Nucleotide sequence of tRNATyr_SP15
SEQ ID NO: 17: Nucleotide sequence of Ec_tRNA-TyrU
SEQ ID NO: 18: Nucleotide sequence of Ec_tRNA-TyrV
SEQ ID NO: 19: Nucleotide sequence of tRNA-Tyr Klebsiella phage VB_KpP_HS106
SEQ ID NO: 20: Nucleotide sequence of ptuA
SEQ ID NO: 21: Nucleotide sequence of ptuB
SEQ ID NO: 22: Amino acid sequence of PtuA
SEQ ID NO: 23: Amino acid sequence of PtuB
SEQ ID NO: 24: Nucleotide sequence of forward primer DRR1
SEQ ID NO: 25: Nucleotide sequence of reverse primer DRR1
SEQ ID NO: 26: Nucleotide sequence of forward primer DRR2
SEQ ID NO: 27: Nucleotide sequence of reverse primer DRR2
SEQ ID NO: 28: Nucleotide sequence of forward primer DRR3
SEQ ID NO: 29: Nucleotide sequence of reverse primer DRR3
SEQ ID NO: 30: Nucleotide sequence of forward primer DRR4
SEQ ID NO: 31: Nucleotide sequence of reverse primer DRR4
SEQ ID NO: 32: Nucleotide sequence of forward primer DRR5
SEQ ID NO: 33: Nucleotide sequence of reverse primer DRR5
SEQ ID NO: 34: Nucleotide sequence of forward primer DRR6
SEQ ID NO: 35: Nucleotide sequence of reverse primer DRR6
SEQ ID NO: 36: Nucleotide sequence of forward primer DRR7
SEQ ID NO: 37: Nucleotide sequence of reverse primer DRR7
SEQ ID NO: 38: Nucleotide sequence of forward primer DRR8
SEQ ID NO: 39: Nucleotide sequence of reverse primer DRR8
SEQ ID NO: 40: Nucleotide sequence of forward primer DRR9
SEQ ID NO: 41: Nucleotide sequence of reverse primer DRR9
SEQ ID NO: 42: Nucleotide sequence of forward primer PDRR1
SEQ ID NO: 43: Nucleotide sequence of reverse primer PDRR1
SEQ ID NO: 44: Nucleotide sequence of forward primer PDRR2
SEQ ID NO: 45: Nucleotide sequence of reverse primer PDRR2
SEQ ID NO: 46: Nucleotide sequence of forward primer PDRR3
SEQ ID NO: 47: Nucleotide sequence of reverse primer PDRR3
SEQ ID NO: 48: Nucleotide sequence of forward primer PDRR4
SEQ ID NO: 49: Nucleotide sequence of reverse primer PDRR4
SEQ ID NO: 50: Nucleotide sequence of forward primer PDRR5
SEQ ID NO: 51: Nucleotide sequence of reverse primer PDRR5
SEQ ID NO: 52: Nucleotide sequence of forward primer PDRR6
SEQ ID NO: 53: Nucleotide sequence of reverse primer PDRR6
SEQ ID NO: 54: Nucleotide sequence of forward primer PDRR7
SEQ ID NO: 55: Nucleotide sequence of reverse primer PDRR7
SEQ ID NO: 56: Nucleotide sequence of forward primer PDRR8
SEQ ID NO: 57: Nucleotide sequence of r reverse primer PDRR8
SEQ ID NO: 58: Nucleotide sequence of forward primer PDRR9
SEQ ID NO: 59: Nucleotide sequence of reverse primer PDRR9
SEQ ID NO: 60: Nucleotide sequence of forward primer ORF75
SEQ ID NO: 61: Nucleotide sequence of reverse primer ORF75
SEQ ID NO: 62: Nucleotide sequence of forward primer pKL-75
SEQ ID NO: 63: Nucleotide sequence of reverse primer pKL-75
SEQ ID NO: 64: Nucleotide sequence of forward primer Rad-T7
SEQ ID NO: 65: Nucleotide sequence of reverse primer Rad-T7
SEQ ID NO: 66: Nucleotide sequence of forward primer T7F1
SEQ ID NO: 67: Nucleotide sequence of reverse primer T7F1-Rad
SEQ ID NO: 68: Nucleotide sequence of forward primer T7F2-Rad
SEQ ID NO: 69: Nucleotide sequence of reverse primer T2F2
SEQ ID NO: 70: Nucleotide sequence of forward primer T7F3
SEQ ID NO: 71: Nucleotide sequence of reverse primer T7F3
SEQ ID NO: 72: Nucleotide sequence of forward primer T7F4
SEQ ID NO: 73: Nucleotide sequence of reverse primer T7F4
SEQ ID NO: 74: Nucleotide sequence of forward primer T7F5
SEQ ID NO: 75: Nucleotide sequence of reverse primer T7F5
SEQ ID NO: 76: Nucleotide sequence of forward primer T7F6
SEQ ID NO: 77: Nucleotide sequence of reverse primer T7F6
SEQ ID NO: 78: Nucleotide sequence of tRNA-Tyr promoter
SEQ ID NO: 79: Nucleotide sequence of tRNA-Tyr promoter
SEQ ID NO: 80: Nucleotide sequence of T5_8e1 phage genome
SEQ ID NO: 81: Nucleotide sequence of T5_8e2 phage genome
SEQ ID NO: 82: Nucleotide sequence of SP15m_8e1 phage genome
SEQ ID NO: 83: Nucleotide sequence of SP15m_8e2 phage genome
SEQ ID NO: 84: Nucleotide sequence of T2_6e1 phage genome
SEQ ID NO: 85: Nucleotide sequence of T2_6e2 phage genome
SEQ ID NO: 86: Nucleotide sequence of SP15m_6e1 phage genome
SEQ ID NO: 87: Nucleotide sequence of SP15m_6e2 phage genome
SEQ ID NO: 88: Nucleotide sequence of T2_6e1 phage genome
SEQ ID NO: 89: Nucleotide sequence of T2_6e2 phage genome
SEQ ID NO: 90: Nucleotide sequence of D15
SEQ ID NO: 91: Amino acid sequence of D15
SEQ ID NO: 92: Nucleotide sequence of DenB
SEQ ID NO: 93: Amino acid sequence of DenB
SEQ ID NO: 94: Nucleotide sequence of forward primer tRNATyr-DRRmSP
SEQ ID NO: 95: Nucleotide sequence of reverse primer tRNATyr-DRRmSP
SEQ ID NO: 96: Nucleotide sequence of forward primer Rad-DRRmSP
SEQ **ID** NO: 97: Nucleotide sequence of reverse primer Rad-DRRmSP
SEQ ID NO: 98: Nucleotide sequence of forward primer DRRSP15 check
SEQ **ID** NO: 99: Nucleotide sequence of reverse primer DRRSP15 check
SEQ ID NO: 100: Nucleotide sequence of tRNA-LysDH10B
SEQ ID NO: 101: Nucleotide sequence of tRNA-AsnDh10B
SEQ ID NO: 102: Nucleotide sequence of tRNA-LysSP15
SEQ ID NO: 103: Nucleotide sequence of tRNA-AsnSP15
SEQ ID NO: 104: Nucleotide sequence of fPrrC170
SEQ ID NO: 105: Nucleotide sequence of rPrrC170m
SEQ ID NO: 106: Nucleotide sequence of fPrrC170m
SEQ ID NO: 107: Nucleotide sequence of rPrrC170
SEQ ID NO: 108: Nucleotide sequence of FpPrrC 170
SEQ ID NO: 109: Nucleotide sequence of RpPrrC170
SEQ ID NO: 110: Nucleotide sequence of fPrrC170-pK
SEQ ID NO: 111: Nucleotide sequence of fPrrC170-pK
SEQ ID NO: 112: Nucleotide sequence of FpK-PrrC170
SEQ ID NO: 113: Nucleotide sequence of RpK-PrrC 170
SEQ **ID** NO: 114: Nucleotide sequence of PrrC170 system sequence
SEQ ID NO: 115: Nucleotide sequence of PrrC170 toxin
SEQ ID NO: 116: Nucleotide sequence of HsdR
SEQ ID NO: 117: Nucleotide sequence of HsdS
SEQ ID NO: 118: Nucleotide sequence of HsdM

## Claims

1. A recombinant phage comprising at least one of genes encoded within a tRNA rich region (TRR) of a T5-like phage.

2. The recombinant phage according to claim 1, wherein the recombinant phage is modified so that at least one of the genes encoded within the TRR is enhanced.

3. The recombinant phage according to claim 1 or 2, wherein at least one of the genes encoded within the TRR is contained in a phage genome.

4. The recombinant phage according to any one of claims 1 to 3, wherein the gene encoded within the TRR is at least one of a gene encoding a Retron inhibitor and a gene encoding a tRNA functioning in a target bacterium.

5. The recombinant phage according to claim 4, wherein the Retron inhibitor is a specific degrading enzyme for an ncRNA in the Retron.

6. The recombinant phage according to claim 4, wherein the gene encoding the Retron inhibitor includes any one selected from nucleotide sequences represented by SEQ ID NO: 5 to SEQ ID NO: 9.

7. The recombinant phage according to claim 6, wherein the Retron inhibitor includes any one selected from amino acid sequences represented by SEQ ID NO: 10 to SEQ ID NO: 14.

8. The recombinant phage according to any one of claims 4 to 7, wherein the tRNA functioning in the target bacterium is a tRNA that inhibits an anti-phage defense system in the target bacterium.

9. The recombinant phage according to any one of claims 4 to 8, wherein the tRNA functioning in the target bacterium is a tRNA to be degraded by an effector protein contained in the anti-phage defense system in the target bacterium.

10. The recombinant phage according to claim 9, wherein the gene encoding the tRNA functioning in the target bacterium includes any one selected from nucleotide sequences represented by SEQ ID NO: 15 to SEQ ID NO: 19.

11. The recombinant phage according to any one of claims 1 to 10, which is a lytic phage.

12. An antibacterial agent or bacteriological testing agent comprising the recombinant phage according to claim 11.

13. A composition comprising the antibacterial agent or the bacteriological testing agent according to claim 12.

14. A sterilization method for killing a target bacterium, the method comprising bringing the composition according to claim 13 into contact with the target bacterium.

15. The sterilization method according to claim 14, wherein the target bacterium is a pathogenic bacterium.

16. The sterilization method according to claim 15, wherein the pathogenic bacterium is at least one selected from Campylobacter jejuni, Vibrio cholerae, Escherichia coli 0157, Legionella pneumophila, Yersinia pestis, Salmonella, and Shigella.

17. A method for preparing a recombinant phage, the method comprising the following steps (1) to (3):
step (1) of preparing the following a) and b):
a) a phage genome; and
b) a nucleotide including at least one of genes encoded within a TRR;
step (2) of obtaining a recombinant phage genome by inserting (b) the nucleotide onto (a) the phage genome;
step (3) of obtaining a recombinant phage into which the recombinant phage genome has been introduced; and
step (4) of collecting the recombinant phage.

18. The method for preparing a recombinant phage according to claim 17, wherein the gene encoded within the TRR is at least one of a gene encoding a Retron inhibitor and a gene encoding a tRNA functioning in a target bacterium.

19. A recombinant phage comprising a gene encoding a protein that is an effector protein constituting the Retron in a bacterium and is a tRNA cleavage enzyme in the bacterium.

20. The recombinant phage according to claim 19, wherein the gene encoding the protein is contained in a phage genome.

21. The recombinant phage according to claim 19 or 20, wherein the gene encoding the effector protein includes at least one of nucleotide sequences represented by SEQ ID NO: 20 and SEQ ID NO: 21.
